# EUROPEAN PATENT SPECIFICATION

(11) **EP 1 619 208 B1**
(45) Date of publication and mention of the grant of the patent: **29.10.2008**
(21) Application number: 04730054.6
(22) Date of filing: 28.04.2004
(51) Int. Cl.: C07K 19/00, C12N 15/62, C12P 21/02, C12P 21/08, A61K 38/16, G01N 33/48, G01N 33/566, G01N 33/68

(54) **CHAPERONINE-TARGET PROTEIN COMPLEX, METHOD OF PRODUCING THE SAME, METHOD OF STABILIZING TARGET PROTEIN, METHOD OF IMMOBILIZING TARGET PROTEIN, METHOD OF ANALYZING THE STRUCTURE OF TARGET PROTEIN, SUSTAINED-RELEASE PREPARATION AND METHOD OF PRODUCING ANTIBODY AGAINST TARGET PROTEIN**
KOMPLEX AUS CHAPERONIN UND TARGETPROTEIN, VERFAHREN ZU DESSEN HERSTELLUNG, VERFAHREN ZUR STABILISIERUNG DES TARGETPROTEINS, VERFAHREN ZUR IMMOBILISIERUNG DES TARGETPROTEINS, VERFAHREN ZUR ANALYSE DER STRUKTUR DES TARGETPROTEINS, ZUBEREITUNG MIT RETARDIERTER FREISETZUNG UND VERFAHREN ZUR HERSTELLUNG VON ANTIKÖRPERN GEGEN DAS TARGETPROTEIN
COMPLEXE DE CHAPERONINE/PROTEINE CIBLE, SON PROCEDE DE PRODUCTION, PROCEDE DE STABILISATION DE PROTEINE CIBLE, PROCEDE D'IMMOBILISATION DE PROTEINE CIBLE, PROCEDE D'ANALYSE DE LA STRUCTURE DE PROTEINE CIBLE, PREPARATION A LIBERATION PROLONGEE ET PROCEDE DE PRODUCTION D'ANTICORPS CONTRE UNE PROTEINE

(30) Priority: 28.04.2003 JP 2003124352
(43) Date of publication of application: 25.01.2006
(73) Proprietor: SEKISUI CHEMICAL CO., LTD., Osaka-shi, Osaka 530-8565 (JP)
(72) Inventor: Ideno, Akira, Sekisui Chemical Co., Ltd., Mishima-gun, Osaka 6188589 (JP); Hata, Jun-ichi, Sekisui Chemical Co., Ltd., Mishima-gun, Osaka 6188589 (JP); Togi, Akiko, Sekisui Chemical Co., Ltd., Mishima-gun, Osaka 6188589 (JP); Furutani, Masahiro, Sekisui Chemical Co., Ltd., Mishima-gun, Osaka 6188589 (JP)
(74) Representative: Schnappauf, Georg
(86) International application number: PCT/JP2004/006189
(87) International publication number: WO 2004/096859

(56) References cited:
- WO-A-01/79259
- WO-A-97/06821
- WO-A-99/42121
- WO-A1-02/052029
- JP-A- 2003 501 064
- KIRSCHNER MARC ET AL: "Transient expression and heat-stress-induced co-aggregation of endogenous and heterologous small heat-stress proteins in tobacco protoplasts" PLANT JOURNAL, vol. 24, no. 3, November 2000 (2000-11), pages 397-411, XP002382494 ISSN: 0960-7412
- ALTAMIRANO M M ET AL: "Oxidative refolding chromatography: folding of the scorpion toxin Cn5" NATURE BIOTECHNOLOGY, NATURE PUBLISHING GROUP, NEW YORK, NY, US, vol. 17, February 1999 (1999-02), pages 187-191, XP002143140 ISSN: 1087-0156
- BRELOER M ET AL: "ISOLATION OF PROCESSED, H-2KB-BINDING OVALBUMIN- DERIVED PEPTIDES ASSOCIATED WITH THE STRESS PROTEINS HSP70 AND GP96" EUROPEAN JOURNAL OF IMMUNOLOGY, WEINHEIM, DE, vol. 28, 1998, pages 1016-1021, XP002928521 ISSN: 0014-2980
- TESHIMA T. ET AL: 'Affinity Purification of Fusion Chaperonin Cpn60-(His)6 from Thermophilic Bacterium Bacillus Strain MS and Its Use in Facilitating Protein Refolding and Preventing Heat Denaturation' BIOTECHNOL. PROG. vol. 16, no. 3, 2000, pages 442 - 446, XP002980896
- ALBERTS, B: ""Molecular Biology of the cell", Chapter II" 28 February 2002 (2002-02-28), * figures 6-84 *

### ANNEX

### SEQUENCE LISTING

<110> Sekisui Chemical Co., Ltd.
<120> CHAPERONINE-TARGET PROTEIN COMPLEX, METHOD OF PRODUCING THE SAME, METHOD OF STABILIZING TARGET PROTEIN, METHOD OF IMMOBILIZING TARGET PROTEIN, METHOD OF ANALYZING THE STRUCTURE OF TARGET PROTEIN, SUSTAINED-RELEASE PREPARATION AND METHOD OF PRODUCING ANTIBODY AGAINST TARGET PROTEIN
<130> 471-4
<140> EP 04 730 054.6
   <141> 2004-04-28
<150> PCT/JP2004/006189
   <151> 2004-04-28
<150> JP2003-124352
   <151> 2003-04-28
<160> 23
<170> PatentIn version 3.1
<210> 1
   <211> 1641
   <212> DNA
   <213> Thermococcus sp. KS-1
<400> 1
   atggcccagc ttgcaggcca gccagttgtt attctacctg agggaactca gaggtacgtt      60
   ggaagggacg cccagaggct caacattctt gctgccagga ttatagccga gacggttaga     120
   accacccttg gaccaaaggg aatggacaag atgctcgttg acagcctcgg cgacatcgtc     180
   atcaccaacg acggtgcaac cattctcgac gagatggaca tccagcaccc tgctgctaag     240
   atgatggttg aggttgctaa gactcaggat aaggaggctg gtgatggtac tactactgcg     300
   gttgttattg ctggtgagct tctgaggaag gctgaggagc ttctcgacca gaacattcac     360
   ccgagcataa tcatcaaggg ctacgccctc gcagcagaga aagcccagga aatactcgac     420
   gagatagcca aggacgttga cgtcgaggac agggagattc tcaagaaggc cgcggtcacc     480
   tccatcaccg gaaaggccgc cgaggaggag agggagtacc tcgctgagat agcagttgag     540
   gccgtcaagc aggttgccga gaaggttggc gagacctaca aggtcgacct cgacaacatc     600
   aagttcgaga agaaggaagg tggaagcgtc aaggacaccc agctcataaa gggtgtcgtc     660
   atcgacaagg aggtcgtcca cccaggcatg ccgaagaggg tcgagggtgc taagatcgcc     720
   ctcatcaacg aggcccttga ggtcaaggag actgagaccg acgccgagat caggatcacc     780
   agcccggagc agctccaggc cttccttgag caggaggaga agatgctcag ggagatggtc     840
   gacaagatca aggaggtcgg cgcgaacgtc gtgttcgtcc agaagggcat tgacgacctt     900
   gcccagcact acctggccaa gtacggcata atggcagtca ggagggtcaa gaagagcgac     960
   atggagaagc tcgccaaggc cactggagct aagatcgtca ccaacgtccg cgacctcacc    1020
   ccggaggacc tcggtgaggc cgagctcgtc gagcagagga aggtcgccgg cgagaacatg    1080
   atcttcgtcg agggctgcaa gaacccgaag gcagtgacaa tactcatcag gggcggtacc    1140
   gagcacgtcg ttgacgaggt cgagagggcc ctcgaggatg ccgtcaaggt cgtcaaggac 1200
   atcgtcgagg acggcaagat cgtcgccgcc ggcggtgctc cggagatcga gctcagcatc 1260
   aggctcgacg agtacgcgaa ggaggtcggc ggcaaggagc agctcgccat cgaggccttt 1320
   gcagaggccc tcaaggtcat tccgaggacc ctcgccgaga acgccggtct cgacccgatc 1380
   gagaccctcg ttaaggtcat cgccgcccac aaggagaagg gaccgaccat cggtgttgac 1440
   gtcttcgagg gcgagccggc cgacatgctc gagcgcggcg tcatcgcccc ggtcagggtt 1500
   ccgaagcagg ccatcaagag cgccagcgag gccgccataa tgatcctcag gatcgacgac 1560
   gtcatcgccg ccagcaagct cgagaaggac aaggagggcg gcaagggcgg tagcgaggac 1620
   ttcggaagcg atctcgactg a 1641
<210> 2
   <211> 1685
   <212> DNA
   <213> Staphylococcus aureus
<400> 2
   tttaaattta attataaata tgattttagt attgcaatac ataattcgtt atattatgat 60
   gactttacaa atacatacag ggggtattaa tttgaaaaag aaaaaaattt attcaattcg 120
   taaactaggt gtaggtattg catctgtaac tttaggtaca ttacttatat ctggtggcgt 180
   aacacctgct gcaaatgctg cgcaacacga tgaagctcaa caaaatgctt tttatcaagt 240
   gttaaatatg cctaacttaa acgctgatca acgtaatggt tttatccaaa gccttaaaga 300
   tgatccaagc caaagtgcta acgttttagg tgaagctcaa aaacttaatg actctcaagc 360
   tccaaaagct gatgcgcaac aaaataagtt caacaaagat caacaaagcg ccttctatga 420
   aatcttgaac atgcctaact taaacgaaga gcaacgcaat ggtttcattc aaagtcttaa 480
   agacgatcca agccaaagca ctaacgtttt aggtgaagct aaaaaattaa acgaatctca 540
   agcaccgaaa gctgacaaca atttcaacaa agaacaacaa aatgctttct atgaaatctt 600
   gaacatgcct aacttgaacg aagaacaacg caatggtttc atccaaagct taaaagatga 660
   cccaagccaa agcgctaacc ttttagcaga agctaaaaag ctaaatgatg cacaagcacc 720
   aaaagctgac aacaaattca acaaagaaca acaaaatgct ttctatgaaa ttttacattt 780
   acctaactta actgaagaac aacgtaacgg cttcatccaa agccttaaag acgatccttc 840
   agtgagcaaa gaaattttag cagaagctaa aaagctaaac gatgctcaag caccaaaaga 900
   ggaagacaac aacaagcctg gtaaagaaga cggcaacaaa cctggtaaag aagacggcaa 960
   caaacctggt aaagaagaca acaaaaaacc tggcaaagaa gacggcaaca aacctggtaa 1020
   agaagacaac aaaaaacctg gcaaagaaga tggcaacaaa cctggtaaag aagacggcaa 1080
   caagcctggt aaagaagatg gcaacaagcc tggtaaagaa gatggcaaca agcctggtaa 1140
   agaagacggc aacggagtac atgtcgttaa acctggtgat acagtaaatg acattgcaaa 1200
   agcaaacggc actactgctg acaaaattgc tgcagataac aaattagctg ataaaaacat 1260
   gatcaaacct ggtcaagaac ttgttgttga taagaagcaa ccagcaaacc atgcagatgc 1320
   taacaaagct caagcattac cagaaactgg tgaagaaaat ccattcatcg gtacaactgt 1380
   atttggtgga ttatcattag cgttaggtgc agcgttatta gctggacgtc cgtcgccgaa 1440
   ctataaaaac aaacaataca caacgataga tatcatttta tccaaaccaa ttttaactta 1500
   tatacgttga ttaacacatt cttatttgaa atgataagaa tcatctaaat gcacgagcaa 1560
   catcttttgt tgctcagtgc attttttatt ttacttactt ttctaaacaa cttctgaaac 1620
   gcctcaacac tttctactct gattacatat acgacatttt tagacattaa aaaatcgact 1680
   ctaga 1685
<210> 3
   <211> 302
   <212> DNA
   <213> Artificial
<220>
   <223> A DNA coding for the s-protein
<400> 3
   atgtcatctt cgaattattg taatcaaatg atgaagtcta gaaacctcac caaggaccgt      60
   tgcaagcccg ttaacacttt tgtgcacgaa tccttagcgg atgtgcaagc cgtttgcagc     120
   caaaaaaacg ttgcatgcaa gaatggccaa acaaactgtt accaatcgta ctcaactatg     180
   tcgatcacag actgcaggga gactggaagc tcaaaatatc caaactgcgc atataaaact     240
   acccaggcaa acaaacacat catcgtcgcg tgtgaaggta acccctatgt cccggttcac     300
   tt                                                                   302
<210> 4
   <211> 45
   <212> DNA
   <213> Artificial
<220>
   <223> A DNA coding for the S-Tag
<400> 4
   aaagaaaccg ctgctgctaa attcgaacgc cagcacatgg acagc 45
<210> 5
   <211> 8
   <212> PRT
   <213> Artificial
<220>
   <223> FLAG peptide
<400> 5
   Asp Tyr Lys Asp Asp Asp Asp Lys
   1               5
<210> 6
   <211> 4
   <212> PRT
   <213> Artificial
<220>
   <223> FLAG peptide
<400> 6
   Asp Tyr Lys Asp
   1
<210> 7
   <211> 9
   <212> PRT
   <213> Artificial
<220>
   <223> FLAG peptide
<400> 7
   Met Asp Phe Lys Asp Asp Asp Asp Lys
   1               5
<210> 8
   <211> 9
   <212> PRT
   <213> Artificial
<220>
   <223> FLAG peptide
<400> 8
   Met Asp Tyr Lys Ala Phe Asp Asn Leu
   1               5
<210> 9
   <211> 96
   <212> DNA
   <213> Artificial
<220>
   <223> Designed oligonucleotide linker
<400> 9
   acccatggga tccctggaag ttctgttcca gggtccgact agtggtggcg gtggctctgt 60
   taaccaccat caccatcacc attaatagct cgaggg 96
<210> 10
   <211> 96
   <212> DNA
   <213> Artificial
<220>
   <223> Designed oligonucleotide linker
<400> 10
   ccctcgagct attaatggtg atggtgatgg tggttaacag agccaccgcc accactagtc 60
   ggaccctgga acagaacttc cagggatccc atgggt 96
<210> 11
   <211> 19
   <212> DNA
   <213> Artificial
<220>
   <223> Designed oligonucleotide primer for PCR
<400> 11
   acagatctat ggcccagct 19
<210> 12
   <211> 18
   <212> DNA
   <213> Artificial
<220>
   <223> Designed oligonucleotide primer for PCR
<400> 12
   acggatccgt cgagatcg 18
<210> 13
   <211> 21
   <212> DNA
   <213> Artificial
<220>
   <223> Designed oligonucleotide primer for PCR
<400> 13
   acactagtat ggcccagctt g 21
<210> 14
   <211> 22
   <212> DNA
   <213> Artificial
<220>
   <223> Designed oligonucleotide primer for PCR
<400> 14
   tggttaactc agtcgagatc gc 22
<210> 15
   <211> 31
   <212> DNA
   <213> Artificial
<220>
   <223> Designed oligonucleotide primer for PCR
<400> 15
   agcggatccg acaacaaatt caacaaagaa c 31
<210> 16
   <211> 31
   <212> DNA
   <213> Artificial
<220>
   <223> Designed oligonucleotide primer for PCR
<400> 16
   tagatctcta ttatactttc ggcgcctgag c 31
<210> 17
   <211> 348
   <212> DNA
   <213> Staphylococcus aureus
<400> 17
   gacaacaaat tcaacaaaga acaacaaaac gcgttctatg agatcttaca tttacctaac 60
   ttaaacgaag aacaacgaaa cgccttcatc caaagtttaa aagatgaccc aagccaaagc 120
   gctaaccttt tagcagaagc taaaaagcta aatgatgctc aggcgccgaa agtagacaac 180
   aaattcaaca aagaacaaca aaacgcgttc tatgagatct tacatttacc taacttaaac 240
   gaagaacaac gaaacgcctt catccaaagt ttaaaagatg acccaagcca aagcgctaac 300
   cttttagcag aagctaaaaa gctaaatgat gctcaggcgc cgaaagta 348
<210> 18
   <211> 23
   <212> DNA
   <213> Artificial
<220>
   <223> Designed oligonucleotide primer for PCR
<400> 18
   acactagtat gtcatcttcg aat 23
<210> 19
   <211> 18
   <212> DNA
   <213> Artificial
<220>
   <223> Designed oligonucleotide primer for PCR
<400> 19 18
   tgcccgggaa gtgaaccg 18
<210> 20
   <211> 21
   <212> DNA
   <213> Artificial
<220>
   <223> Designed oligonucleotide primer for PCR
<400> 20
   acgcatgcta atacgactca c 21
<210> 21
   <211> 22
   <212> DNA
   <213> Artificial
<220>
   <223> Designed oligonucleotide primer for PCR
<400> 21
   acctcgaggt tgtacagttc at 22
<210> 22
   <211> 51
   <212> DNA
   <213> Artificial
<220>
   <223> A DNA coding for the S-Tag (sense)
<400> 22
   tcgagaaaga aaccgctgct gctaaattcg aacgccagca catggacagc g 51
<210> 23
   <211> 51
   <212> DNA
   <213> Artificial
<220>
   <223> A DNA coding for the S-Tag (antisense)
<400> 23
   gatccgctgt ccatgtgctg gcgttcgaat ttagcagcag cggtttcttt c 51

## Description

### TECHNICAL FIELD

The present invention relates to a chaperonin-target protein complex and a method of producing the same, and a method of stabilizing the target protein, a method of immobilizing the target protein, a method of analyzing the structure of the target protein, a sustained-release formulation, and a method of producing an antibody against the target protein. More particularly, it relates to a chaperonin-target protein complex wherein a target protein is bound to a chaperonin via an affinity tag and a method of producing the same, and a method of stabilizing the target protein, a method of immobilizing the target protein, a method of analyzing the structure of the target protein, a sustained-release formulation, and a method of producing an antibody against the target protein, each using the chaperonin-target protein complex.

### BACKGROUND ART

An analysis of complete genome sequence of various organisms including human, mouse, and yeast has been completed, and it is considered that a trend of research in biotechnology is making the progress from a gene analysis towards a comprehensive research of proteins. As a consequence of such research, it is expected that discovery of proteins relating to diseases and unraveling of interaction between proteins in vivo leads to a development of a new drug. Recently, research tools such as a two-hybrid method for detection of interaction between proteins and protein chips for detection of protein expression have been developed and widely used. Up to now, though DNA chips have been used for screening genes relating to diseases, gene translation has not always correlated well with the protein expression. Thus, a research tool using proteins as a sample including protein chips draws attention as a new alternative method.

Proteins must be handled with attention to protect the proteins from denaturation. One of important factors by which proteins are characterized is its higher-ordered structure. However, changes in the external environment such as heat or pH break the higher-ordered structure of proteins with the consequence of detraction of an activity of the protein. Problems for industrial application of proteins are how to maintain its higher-ordered structure and to prevent its denaturation. For example, in the case of application of proteins immobilized on a polystyrene carrier or support or the like, it must be noted not to have a higher-ordered structure of the proteins be changed due to hydrophobic interaction involved with immobilization on the carrier or the like. Immobilization of proteins on a carrier has another problem such that an active site of the proteins faces the carrier, resulting in preventing its activity from functioning properly. Therefore, a controlling method for immobilization of proteins on a carrier with keeping their orientation has been also desired.

On the other hand, a certain type of proteins is brought to attention in application as a probe for screening a new drug. More specifically, some proteins in cells are bound specifically to different types of physiologically active substances, thereby relating to a transmission of its action. An agonist and an antagonist that act against them can be candidate substances for a new drug for controlling transmission of the action, so that identification of the proteins and their stereostructures draw considerable concern. Though there are NMR Method and X-ray crystallographic analysis to analyze stereostructures of proteins, it is generally difficult for NMR Method to analyze proteins with a molecular weight of 50 kDa and more. As for the other method, X-ray crystallographic analysis, there is no limitation about a molecular weight of proteins, but this method necessitates consideration of various conditions for crystallization on each protein, resulting in being rate-limiting for high-throughput analysis, which is viewed with suspicion. In proteins, as just described, their tertiary structure and quaternary structure are very important for determination of their nature and quality.

Recently, it is further brought to attention that an improvement of a recombinant producing technology of proteins allows physiologically active proteins such as cytokines and proteases to be produced in a large amount to apply as a new drug. However, many of these proteins are decomposed by proteases, resulting in an extremely short residence time in vivo. It is considered that most of decomposed proteins as just described include a protein consisting of a functional domain of physiologically active proteins or a protein having different structures from that of native ones, resulting in being vulnerable to protease destruction. Such an effort as embedding physiologically active proteins with water-soluble polymers is made to lengthen the residence time in vivo, but problems involving a cumbersome and complicated process remain, so that a simpler method has been required.

An antibody drug is also brought to attention as one of protein drugs. In order to obtain an antibody capable of being a drug, it is necessary to immunize an animal with an antigen causing a disease, to recover antibodies, its genes, and hybridomas, and to assess and improve them. However, in the case that the antigen is easy to be decomposed in the blood of the inoculated animals, it is impossible to induce a sufficient immune response, and might not obtain a target antibody.

As described, proteins have unlimited potential, but a method by which the proteins are handled and controlled more easily and more efficient has been widely desired.

WO 99/42121 discloses a method of purifying secreteable heat shock proteins comprising an affinity tag.

Kirschner Marc et al. Plant Journal, vol. 24, no. 3, November 2000 (2000-11), pages 397-411, discloses plant heat shock proteins of the hsp20 chaperone family fused to affinity tags.

WO 97/06821 discloses immunogenic compositions comprising at least one heat shock protein and one or more hybrid target antigens having a heat shock protein binding domain.

WO 01/79259 discloses a complex comprising a heat shock protein and one or more epitopes covalently fused to a tethering molecule (called "javelin") having affinity to said heat shock protein.

It is therefore an object of the present invention made in view of the problems and drawbacks described above to provide a chaperonin-target protein complex by which the target protein is handled more easily and a method of producing the same, and a method of stabilizing the target protein, a method of immobilizing the target protein, a method of analyzing the structure of the target protein, a sustained-release formulation, and a method of producing an antibody against the target protein, each using the chaperonin-target protein complex.

### DISCLOSURE OF THE INVENTION

The present invention proposed for achieving the aim described above is to accommodate and hold a target protein via an affinity tag in a quaternary structure of a chaperonin protein (also referred to as a heat-shock protein 60 kDa or a thermosome).

More specifically, an aspect of the present invention relates to:
(1) A chaperonin-target protein complex including a fusion protein including a chaperonin subunit and an affinity tag linked to the chaperonin subunit via a peptide bond, and a target protein for which the affinity tag shows a specific affinity, wherein the target protein is bound to the affinity tag by means of the specific affinity, thereby forming a chaperonin ring structure consisting of a plurality of chaperonin subunits and wherein the target protein is accommodated in the chaperonin ring structure of the fusion protein;
(2) The chaperonin-target protein complex as described in (1) wherein the fusion protein includes one chaperonin subunit, and wherein the affinity tag is linked via the peptide bond to the N-terminus and/or the C-terminus of the chaperonin subunit;
(3) The chaperonin-target protein complex as described in (1) wherein the fusion protein includes a chaperonin subunit linkage composed of 2 to 20 chaperonin subunits serially linked to one another, and wherein the affinity tag is linked to at least one site selected from a group consisting of the N-terminus of the chaperonin subunit linkage, the C-terminus of the chaperonin subunit linkage, and a linking site of the chaperonin subunits;
(4) The chaperonin-target protein complex as described in (1), (2), or (3) wherein the ratio of the number of the chaperonin subunits to the number of the affinity tag is in the range of from 1 : 2 to 9 : 1;
(5) The chaperonin-target protein complex as described in (3) or (4) that is provided with a sequence to be cleaved by a site-specific protease between the subunits of the chaperonin subunit linkage;
(6) The chaperonin-target protein complex as described in one of (1)-(5) having two chaperonin rings non-covalently associated on each other's ring plane so as to form a two-layer chaperonin ring structure; and
(7) The chaperonin-target protein complex as described in (6) having two chaperonin rings non-covalently associated on each other's ring plane or on each other's side so as to form a fibrous chaperonin ring structure.
   Another aspect of the present invention relates to:
(8) The chaperonin-target protein complex as described in one of (1)-(7) wherein the living thing from which the chaperonin is derived is one selected from a group consisting of bacteria, archaea, and eukaryotes;
(9) The chaperonin-target protein complex as described in one of (1)-(8) wherein the affinity tag is one selected from a group consisting of an antibody, streptoavidin, protein A, protein G, protein L, S peptide, S-protein, and a partial fragment thereof;
(10) The chaperonin-target protein complex as described in one of (1)-(9) wherein the target protein is a polypeptide including a fragment of six or more amino acid residues, and includes a partner tag having a specific affinity for the affinity tag;
(11) The chaperonin-target protein complex as described in one of (1)-(10) wherein the target protein is one selected from a group consisting of a heavy chain of an antibody, a light chain of an antibody, and a polypeptide including a fragment of 6 or more amino acid residues thereof, and includes a partner tag having a specific affinity for the affinity tag;
(12) The chaperonin-target protein complex as described in one of (1)-(10) wherein the target protein includes one selected from a group consisting of a virus antigen, a seven transmembrane receptor, a cytokine, a protein kinase, a phosphoprotein phosphatase, and a partial fragment thereof;
(13) A method of producing the chaperonin-target protein complex as described in one of (1)-(12) the method including steps of mixing the fusion protein composed of the chaperonin subunit and the affinity tag with the target protein including the partner tag having an affinity for the affinity tag, and binding the fusion protein to the target protein by means of a specific affinity between the affinity tag and the partner tag;
(14) A method of producing the chaperonin-target protein complex as described in one of (1)-(12) wherein the partner tag having a specific affinity for the affinity tag is a polypeptide, and the method including a step of transcribing and translating in the same host both of a gene containing a gene encoding the chaperonin subunit and a gene encoding the affinity tag and a gene containing a gene encoding the target protein and a gene encoding the partner tag, thereby binding the target protein to the chaperonin fusion protein;
(15) A method of producing the chaperonin-target protein complex as described in one of (1)-(12) wherein the partner tag having a specific affinity for the affinity tag is a polypeptide, and the method including a step of transcribing and translating in the same host three genes consisting of a gene containing a gene encoding the chaperonin subunit and a gene encoding the affinity tag, a gene containing a gene encoding the target protein and a gene encoding the partner tag, and a gene encoding the chaperonin subunit or its linkage, thereby binding the target protein, the chaperonin fusion protein, and the chaperonin subunit or its linkage; and
(16) The method as described in (14) or (15) wherein the protein complex is produced in a host selected from a group consisting of bacteria, yeasts, animal cells, plant cells, insect cells, animals, plants, and insects.
   Still another aspect of the present invention relates to:
(17) The method as described in (14) or (15) wherein the protein complex is produced in a cell-free translation system;
(18) A method of stabilizing the target protein, the method including a step of binding the target protein via the affinity tag to the fusion protein included in the chaperonin-target protein complex as described in one of (1)-(9) to have the target protein be accommodated in the chaperonin ring structure;
(19) A method of immobilizing the target protein, the method including steps of immobilizing on a carrier for immobilization the fusion protein included in the chaperonin-target protein complex as described in one of (1)-(9), and binding the target protein via the affinity tag to the fusion protein to have the target protein be accommodated in the chaperonin ring.
(20) A method of analyzing the structure of the target protein, the method including steps of crystallizing a protein complex obtained by accommodating the target protein bound via the affinity tag to the fusion protein included in the chaperonin-target protein complex as described in one of (1)-(9), and obtaining information about three-dimensional structure of the target protein from an X-ray diffraction image obtained by irradiating a crystal obtained by the crystallizing;
(21) A sustained-release formulation for a physiologically active protein or a low molecular weight drug wherein the physiologically active protein or the low molecular weight drug is accommodated in the fusion protein included in the chaperonin-target protein complex as described in one of (1)-(9) via the partner tag for the affinity tag;
(22) The sustained-release formulation as described in (21) wherein the chaperonin is derived from human; and
(23) A method of producing an antibody against a target antigen protein, the method including steps of binding the target antigen protein to the fusion protein included in the chaperonin-target protein complex as described in one of (1)-(9), and immunizing an animal therewith as an immunogen.

The chaperonin-target protein complex in the invention performs more readily stabilization of the target protein, immobilization of the target protein on a carrier, a structural analysis of the target protein, formulation of the sustained-release target protein, and production of an antibody against the target protein.

According to the method of stabilizing the target protein in the invention, the target protein is stabilized more readily.

According to the method of immobilizing of the target protein in the invention, the target protein is arranged in order, thereby showing a characteristic of the target protein more efficiently.

According to the method of analyzing the structure of the target protein in the invention, the X-ray analysis is performed readily enough to dispense with consideration of a condition for crystallization depending on the nature of the target protein.

According to the sustained-release formulation in the invention, the target protein is not decomposed rapidly in the blood to exert the drug efficacy at a specific affected area.

According to the method of producing an antibody in the invention, the target protein is not decomposed rapidly in the blood of the immunized animal to carry out immune response more efficiently.

### BRIEF DESCRIPTION OF THE DRAWINGS

Fig. 1 is a schematic illustration of the stereostructure of Escherichia coli chaperonin (GroEL);
Fig. 2A is a schematic illustration showing a state wherein two fusion proteins each composed of a chaperonin subunit 4-times linkage and an affinity tag form a ring structure;
Fig. 2B is a schematic illustration of a fusion protein composed of a chaperonin subunit 4-times linkage and an affinity tag;
Fig. 3A is a conceptual scheme showing a state that a chaperonin linkage is immobilized on a carrier;
Fig. 3B is a conceptual scheme showing a state that a chaperonin-affinity tag fusion protein is immobilized on a carrier;
Fig. 3C is a conceptual scheme showing a state that a target protein is immobilized on a carrier via a chaperonin-affinity tag fusion protein;
Fig. 4 is an illustration showing constitution of a main part of an expression vector pETD₂ (TCPβ)₈;
Fig. 5 is a photograph of a fusion protein composed of an archaeal chaperonin having eight subunits and a protein A under a transmission electron microscope;
Fig. 6 is a photograph of a chaperonin-monoclonal antibody complex obtained by acting a monoclonal antibody on a fusion protein composed of an archaeal chaperonin having eight subunits and a protein A under a transmission electron microscope;
Fig. 7 is a photograph of a chaperonin-GFP complex obtained by acting a GFP-S tag on a fusion protein composed of an archaeal chaperonin having eight subunits and a protein A under a transmission electron microscope;
Fig. 8A is a graph for comparing an antibody activity of a mouse monoclonal antibody immobilized on a carrier;
Fig. 8B is a graph for comparing a heat stability of an immobilized mouse monoclonal antibody.

### DESCRIPTION OF THE PREFERRED EMBODIMENTS

A chaperonin-target protein complex in the present invention includes a fusion protein in which an affinity tag is linked a chaperonin subunit via a peptide bond and a target protein for which the affinity tag shows a specific affinity, wherein the target protein is bound to the affinity tag by means of the specific affinity, thereby forming a chaperonin ring structure consisting or a plurality of chaperonin subunits and wherein the target protein is accommodated in the chaperonin ring structure of the fusion protein.

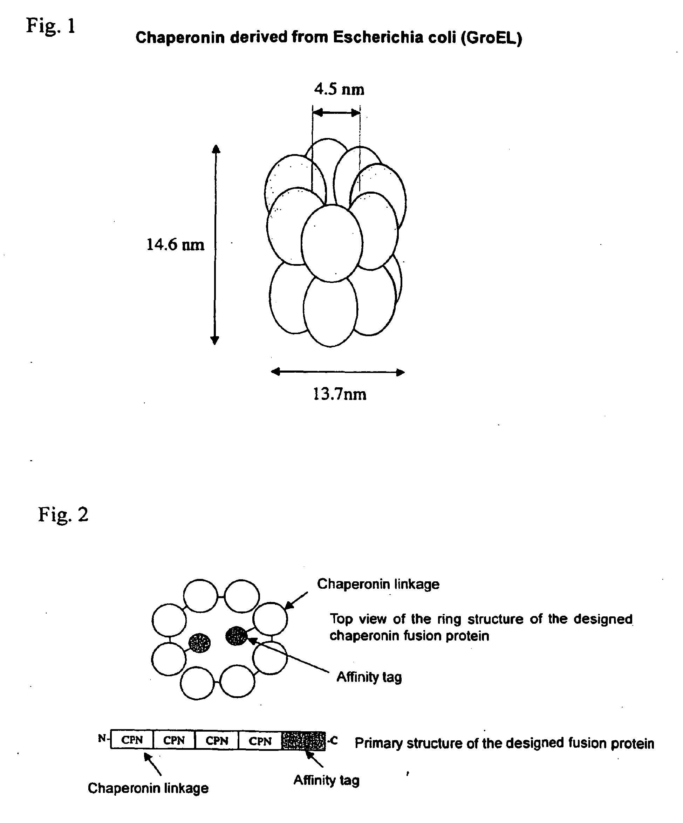

A native chaperonin, one of molecular chaperones that assist protein folding, has a two-layer structure formed by two rings (chaperonin rings) each having seven to nine subunits with a molecular weight of about 60 kDa, being a huge protein with a molecular weight of 1,000 kDa. The chaperonin is found in every living thing such as bacteria, archaea, and eukaryotes, and assists protein folding and contributes to structure stabilization in the presence or absence of an energy substance ATP in cells. Escherichia coli chaperonin (GroEL), for example, has a cavity with an inner diameter of 4.5 nm and a height of 14.5 nm in each layer of a two-layer ring. (see Fig. 1) The cavity of the one-layer chaperonin ring has a space in which a globular protein with a molecular weight of 60 kDa is sufficiently accommodated. It is well known that the chaperonin complex functions in transiently accommodating folded intermediates of various proteins or denatured proteins in the cavity, and once a folded structure of the protein is formed, the complex is conjugated with ATP decomposition to release the accommodated protein from the cavity.

In the present invention, a fusion protein (hereinafter referred to as a chaperonin-affinity tag fusion protein or simply a chaperonin fusion protein) in which a polypeptide affinity tag is linked to the chaperonin subunit via the peptide bond is produced to form the ring structure by assembling the chaperonin subunits. At this time, the affinity tag linked to the chaperonin is preferably accommodated in the chaperonin ring. The target protein needs to include a partner tag having a specific affinity for the affinity tag. The partner tag is bound to the affinity tag, thereby ensuring that the target protein is accommodated in the chaperonin ring (hereinafter the resulting complex composed of the chaperonin and the target protein is referred to as a chaperonin-target protein complex or simply a chaperonin complex).

Herein, the "affinity tag" and the "partner tag" are terms used to designate two kinds of substances having a specific affinity for each other such as a hormone and a receptor, an antigen and an antibody, and an enzyme and a substrate that are used as tags, referring to one as an affinity tag and to the other as a partner tag. Specifically, the chaperonin-target protein complex in the present invention applies either of the tags to be linked to the chaperonin subunit to form the fusion protein, and one fused with the chaperonin subunit is referred to as an affinity tag and the other is referred to as a partner tag. A specific affinity between the affinity tag and the partner tag is composed of a noncovalent interaction such as a hydrogen bond, a hydrophobic interaction, and an intermolecular force.

As to a combination of the affinity tag and the partner tag, any combination may be applied if the combination has an affinity by means of a specific interaction between the two tags. However, the affinity tag is preferably made of a polypeptide since it needs to be expressed as a fusion protein with the chaperonin subunit.

A combination of protein A or protein G and Fc region of IgG is taken for an example of the combination of the affinity tag and the partner tag. The protein A (Bridigo, M. M., J. Basic. Microbiology, 31, 337, 1991) consisting of about 470 amino acid residues and derived from Bacillus sp. or Staphylococcus sp. is well known and often used for purification of IgG from anti-sera. IgG binding domain of the protein A derived from Staphylococcus aureus consists of about 60 amino acid residues (Saito, A., Protein Eng. 2, 481, 1989). The protein G consisting of about 450 amino acid residues and derived from Streptococcus sp. (Frahnestock, S. R., J. Bacteriol. 167, 870, 1986) is well known and often used for purification of IgG as well as the protein A. It has already shown that a binding region with IgG consists of about 50 to 60 amino acids (Gronenborn, A. M., Science, 253, 657,1991).

Another example of the combination of the affinity tag and the partner tag includes a combination of protein L and immunoglobulin κ light chains. The protein L is a protein bound specifically to κ light chains of various immunoglobulin classes such as IgG, IgM, IgA, IgE, and IgD or their subclasses without interfering with their antigen binding site. The protein L that is known up to now consists of about 720 amino acid residues and is derived from Finegoldia magna (Kastern, W., Infect. Immun. 58, 1217, 1990). A binding domain with κ light chain consisting of about 80 amino acids has already being studied in detail (Wikstron, M., Biochemistry 32, 3381, 1993).

Still another example of a combination of the affinity tag and the partner tag includes a combination of S protein and S peptide. It is known that the S protein and the S peptide, both of which are a part of ribonucleases, are bound each other so as to show a ribonuclease activity. They also have a high specific affinity for each other, so as to be often used for purification of proteins. In the S peptide, the S-tag consisting of 15 amino acid residues is used for research. SEQ ID No. 3 designates a DNA sequence encoding the S protein and SEQ ID No. 4 designates a DNA sequence encoding the S-Tag.

Yet another example of a combination of the affinity tag and the partner tag includes a combination of a FLAG peptide and an antibody that uses the FLAG peptide as an epitope. The FLAG peptide is often used for purification of proteins. For example, the FLAG peptide consisting of 11 amino acids is fused to the N-terminus of a yeast transcription factor, thereby purifying a fusion protein via its antibodies (Witzgall, R., Biochem., 223, 291, 1994). Examples of the FLAG peptides for general application include a peptide consisting of DYKDDDDK (SEQ ID No.5), a peptide consisting of DYKD (SEQ ID No. 6), a peptide consisting of MDFKDDDDK (SEQ ID No. 7), and a peptide consisting of MDYKAFDNL (SEQ ID No. 8). These peptides respectively show an affinity for M1 monoclonal antibody, M5 monoclonal antibody, and M2 monoclonal antibody. These antibodies are available from a reagent maker such as Sigma.

The affinity tag making up the chaperonin-target protein complex in the present invention may use not only the full-length of the above-mentioned protein A, protein G, protein L, S peptide, S-protein, and the like, but also their fragments including a binding region with the partner tag. Further, the above-mentioned combinations of the affinity tag and the partner tag may be reverse combinations therefrom. For example, Fc region of IgG as the affinity tag is expressed as a fusion protein with the chaperonin, while protein A or protein G as the partner tag is expressed as a fusion protein with the target protein. The chaperonin-target protein complex can be prepared by means of the specific affinity between the affinity tag and the partner tag.

The present invention makes use of not only the specific affinity between the above-mentioned peptides, but also a specific affinity between the peptide and a low molecular weight compound. An affinity between streptoavidin and biotin is cited as an example. More specifically, the full-length of the streptoavidin or a partial fragment showing a biotin binding activity selected as the affinity tag is linked to the chaperonin subunit via a peptide bond, so as to produce a chaperonin-affinity tag fusion protein, whereas the biotin as the partner tag is linked to the target protein. Herein, the biotin is readily linked by use of a kit on the market. In this way, the target protein is accommodated in the chaperonin ring by means of a specific affinity between the biotin and the streptoavidin.

The partner tag may be a part of the target protein or a different substance from the target protein. The former example includes such a combination that the affinity tag is protein A, the target protein is IgG, and the partner tag is Fc region of IgG. The latter example includes such a combination that the affinity tag is S-protein, the partner tag is S peptide, and the target protein is linked to S peptide.

The chaperonin making up the chaperonin-target protein complex in the present invention may naturally be a fusion protein in which the affinity tag is fused to one chaperonin subunit, but also a fusion protein that includes a chaperonin subunit linkage (viz., chaperonin linkage) composed of 2 to 20 chaperonin subunits serially linked to one another, and in which the affinity tag is fused via a peptide bond to at least one site selected from a group consisting of the N-terminus of the chaperonin subunit linkage, the C-terminus of the chaperonin subunit linkage, and a linking site of the chaperonin subunits. According to the structure of the chaperonin revealed by X-ray crystallographic analysis, the structure is highly flexible with both the N-terminus and the C-terminus of the chaperonin located at the side of the cavity. In particular, at least 20 amino acids of the C-terminus show a highly flexible structure (George, Cell 100, 561, 2000). Consequently, the chaperonin subunit linkage with the C-terminus of one chaperonin subunit and the N-terminus of the other chaperonin subunit linked via a polypeptide having an appropriate length forms a chaperonin ring structure as well as monomer subunits.

The ratio of the number of the chaperonin and the number of the affinity tag may be in the range of from 1 : 2 to 20 : 1, preferably from 1 : 2 to 9 : 1. If the number is higher than this range, formation of the ring structure may be made difficult. In the present invention, if the ability of a chaperonin to be self-assembled into a ring structure is maintained, not only a wild-type chaperonin but also a chaperonin with an amino acid mutant can also be used.

The chaperonin-target protein complex in the present invention not only provides a space separated from the external environment, but also functions in protein folding, thereby enabling to fold the protein normally and simultaneously to be expected to stabilize the structure of the protein. The protein folding reaction of the chaperonin complex with a substrate protein as a single polypeptide occurs usually in the ratio of 1 : 1. Consequently, in the present invention, the fusion protein is designed preferably such that one molecule of the affinity tag is accommodated in the chaperonin ring or the chaperonin complex, in order to express the folding function of the chaperonin. However, depending on the molecular weight of the tag, the tag can be correctly folded even if two or more molecules are accommodated.

The chaperonin making up the chaperonin-target protein complex in the present invention may be derived from bacteria, archaea, and eukaryotes. However, formation of the structure of the chaperonin is varied depending on the living thing from which the chaperonin is derived. For example, the number of chaperonin subunits constituting a chaperonin ring is seven in the case of chaperonin derived from bacteria, while the number of that is eight to nine in the case of chaperonin derived from archaea, while the number of that is eight in the case of chaperonin derived from eukaryotes. In the present invention, the ratio of the number of chaperonin subunits to the number of affinity tags in the fusion proteins is selected preferably depending on the origin of a chaperonin used. Specifically, when a bacterial chaperonin is used, a fusion protein wherein the number of chaperonin subunits : the number of affinity tags is 7 : 1 is preferable for easy formation of a higher-ordered structure of the chaperonin, and when an archaeal chaperonin wherein the number of subunits constituting chaperonin ring is eight is used, a fusion protein wherein the number of chaperonin subunits : the number of affinity tags is 1 : 1, 2 : 1, 4 : 1, or 8 : 1 is preferable for easy formation of a higher-ordered structure of the chaperonin. For example, when an affinity tag is expressed in a fusion protein using an archaeal chaperonin wherein the subunits have formed a linkage having eight subunits linked therein, one molecule of the chaperonin subunit 8-times linkage forms a ring structure and accommodates the affinity tag in its cavity. An example taken as an archaeal chaperonin and its chaperonin subunits includes a chaperonin derived from Thermococcus sp. strain KS-1 (TCP) and a chaperonin β subunit (TCPβ) that is a kind of its subunits. In short, TCP is a complex composed of eight TCPβ. A chaperonin β subunit 8-times linkage ((TCPβ)₈) having eight TCPβ linked serially forms a complex having a ring structure as well as a native TCP.

A fusion protein including a chaperonin subunit and an affinity tag linked to the chaperonin subunit is produced in a conventional host-vector expression system. For example, in order to prepare a fusion protein wherein the number of chaperonin subunits : the number of affinity tags is 2 : 1, a fusion gene in which one affinity tag gene is linked to two chaperonin genes is prepared, and then transcribed and translated. More specifically, one gene encoding a chaperonin subunit is linked to a downstream of a promoter, whereupon a gene encoding one chaperonin in which a codon frame is designed so as to be in the same open reading frame is arranged at a further downstream thereof. At this time, when these chaperonin genes are translated in the same open reading frame, DNA sequence that becomes an appropriate polypeptide linker consisting of about 10 to 30 amino acids is preferably arranged between these genes. As well, at a further downstream thereof, a gene encoding a polypeptide linker that a codon frame is designed so as to be in the same open reading frame and a gene encoding an affinity tag are arranged. In such an arrangement, a fusion protein wherein the number of chaperonin subunits : the number of affinity tags is 2 : 1 is prepared. Fusion proteins with other ratios are prepared in the same way, and as described above, the affinity tag may be arranged at any site selected from a group consisting of the N-terminus of the chaperonin subunit linkage, the C-terminus of the chaperonin subunit linkage, and a linking site between the chaperonin subunits, and may further be arranged at two or more sites.

A digestion site of a site-specific protease may be arranged at a peptide linker site between the chaperonin subunits or at a linking site between the chaperonin and the affinity tag. Examples of proteases include restriction proteases such as a thrombin, an enterokinase, and an activated blood coagulation factor X.

The fusion protein expressed by the above-mentioned way forms a ring structure and further forms a two-layer structure non-covalently associated on each other's ring plane, thus being stabilized. In the case of fusion expression of the affinity tag and an archaeal chaperonin subunit 4-times linkage, two molecules of the chaperonin subunit 4-times linkage form a ring structure and accommodates the affinity tag in its cavity. Its schematic illustration is shown in Fig. 2. In Fig. 2, a white circle denotes the chaperonin subunit, a black circle denotes the target protein, a straight line connecting the white circles or the black circle and the white circle denotes the peptide bond including a linkage by the peptide linker. "CPN" in Fig. 2 denotes the chaperonin subunit. There is also the case wherein other ratios are suitable depending on a form or a molecular weight of the target protein. For example, even if the number of chaperonin derived from E. coli : the number of target protein is 3 : 1, the fusion protein can be associated to form a ring structure consisting of two or three molecules of the fusion protein.

When the chaperonin is present at a high concentration of not less than 1 mg/mL and in the presence of a Mg-ATP, two-layer chaperonin rings may further be bound reversibly to one another on each other's ring plane to assemble into a fibrous structure (Trent, J. D., et al., 1997, Proc. Natl. Acad. Sci. U.S.A. 94, 5383-5388: Furutani, M. et al., 1998, J. Biol. Chem. 273, 28399-28407). Because the fusion protein in the present invention is expressed at a high concentration in the living body, the protein may assemble into a fibrous structure. Even if the fusion protein assembles into a fibrous structure, the structure can be dissociated into each two-layer ring structure by dilution. Such a fusion protein assembling into a fibrous structure is also one aspect of the present invention.

The invention features a target protein accommodated and held in a chaperonin ring structure by means of an affinity between an affinity tag and a partner tag. The target protein used in this invention is not particularly limited, and examples thereof include full-length proteins of immunoglobulins such as IgG, IgM, IgA, IgE, and IgD or proteins including fragments thereof. When the target protein is IgG, protein A or protein G is used as the affinity tag to which a chaperonin is fused to give a fusion protein. Fc region of IgG has a specific affinity for the protein A or the like, via which the target protein, IgG, is accommodated in the chaperonin ring. When the target protein is Fab or scFv (viz. single chain Fv) both which are partial fragments of IgG, protein L is used as the affinity tag, via which the target protein is accommodated in the chaperonin ring on the same principle.

Recently, a phage library technique for screening a polypeptide specifically bound to a desired antigen and its gene by displaying a random sequence on a phage structural protein has gotten attention (Kumagai et al., PROTEIN, NUCLEIC ACID AND ENZYME, 1998, 43, 159-167). There is such a similar technique as screening Escherichia coli or a polypeptide bound to a target antigen and by displaying a random sequence of amino acids on a flagellar protein in the Escherichia coli. The polypeptide obtained from these methods expects an industrial applicability as an antibody mimic. In the present invention, any peptide having physiological activities such as antigen binding ability is adaptable as the target protein. The target protein is preferably a polypeptide consisting of six or more amino acid residues to maintain a target activity.

Other target proteins to be accommodated in a chaperonin ring include proteins encoded in a genome of pathogenic viruses such as hepatitis B virus, hepatitis C virus, HIV, or influenza virus (coat protein, core protein, protease, reverse transcriptase, integrase, and the like), seven transmembrane receptor protein (G protein-coupled receptor), proteins belonging to growth factors such as platelet growth factor, hematopoietic stem cell growth factor, hepatocyte growth factor, transforming growth factor, nerve growth-trophic factor, fibroblast growth factor, and insulin-like growth factor, and proteins such as tumor necrosis factor, interferon, interleukin, erythropoietin, granulocyte-colony stimulating factor, macrophage-colony stimulating factor, albumin, human growth hormone, protein kinase, and phosphoprotein phosphatase. The target protein may further be any disease-related gene products derived from higher animals such as human and mouse. Any enzymes and proteins useful in chemical processes, food processing, and other industries can be also the target protein constituting the chaperonin-target protein complex in the invention.

As for a method of producing the chaperonin-target protein complex in the present invention, it is only necessary to mix in vitro the chaperonin fusion protein with the target protein including the partner tag in an appropriate biochemical buffer. That forms the chaperonin-target protein complex via the affinity tag included in the chaperonin fusion protein and the partner tag included in the target protein. Especially, it is preferable that a factor relating to stabilization of the chaperonin such as magnesium ion, potassium ion, and nucleotide triphosphate including ATP is added in a reaction solution. Further, the individual chaperonin subunit or the chaperonin subunit linkage that is not fused to the affinity tag may be added to form the chaperonin-target protein complex.

It is also possible to prepare the chaperonin-target protein complex in vivo by means of a host-vector expression system. More specifically, transcription and translation (viz., co-expression) of both a gene encoding the chaperonin-affinity tag fusion protein and a gene containing a gene encoding the target protein and a gene encoding the partner tag in the same host bind the target protein to the affinity tag by means of a specific affinity for the partner tag, thereby forming the chaperonin-target protein complex in the host. Herein, the gene encoding the chaperonin-affinity tag fusion protein and the gene encoding the target protein may be arranged under the control of the same promoter or may be expressed under the control of different promoters. Under the control of the different promoters, both expression units may be arranged in the same plasmid or in different plasmids.

In arrangement on the different plasmids, in the case of using Escherichia coli, for example, as the host, it is only necessary to introduce one unit into a downstream of an expression promoter on a P15A plasmid such as pACYC184 and the other unit into an expression vector having a colE1 DNA replication starting region such as pET. Since the plasmids of the two are capable of coexistence in the same host, the both genes coexist in the host only by getting each plasmid to have a different drug resistance marker. The both genes are expressed under the control of the respective promoters. A plasmid whose replication site is derived from R6K (Kolter, R., Plasmid 1, 157, 1978) may be applicable, since being capable of coexistence with the above-mentioned P15A plasmid or colE1 plasmid in the host.

As for another method of preparing the chaperonin-target protein complex in vivo, simultaneous expression of three genes including a gene encoding only an individual chaperonin subunit or the chaperonin subunit linkage as well as both the gene encoding the chaperonin-affinity tag fusion protein and the gene containing the gene encoding the target protein and the gene encoding the partner tag as described above associates three of these consisting of the chaperonin-affinity tag fusion protein, the individual chaperonin subunit or the chaperonin subunit linkage, and the target protein, thereby forming the chaperonin-target protein complex in the host. This method lessens the number of the chaperonin subunits in the chaperonin-affinity tag fusion protein, with the consequence that a molecular weight of the chaperonin-affinity tag fusion protein is lessened. Thus, the chaperonin-affinity tag fusion protein is expressed more easily, so that the chaperonin-target protein complex is produced more efficiently.

Three genes consisting of the gene encoding the chaperonin-affinity tag fusion protein, the gene encoding the chaperonin linkage, and the gene encoding the target protein may be arranged under the control of the same promoter, or may be expressed under the control of different promoters. Further, as described above, they may be arranged in two or more plasmids capable of coexistence in the same host. Preparation of these plasmids is readily designed and produced by means of a normal genetic engineering.

Generally, when the size of an expression plasmid is 10 kb or more, the copy number may be decreased in E. coli and the like, resulting in a reduction in the amount of the chaperonin fusion protein expressed. For example, when a chaperonin fusion protein having a chaperonin 8-times linkage is produced, the size of an expression plasmid therefor is 15 kbp or more. As a countermeasure, the same gene expressing the same chaperonin fusion protein is introduced into two vectors having different replication regions and different drug resistant genes, and E. coli or the like is transformed with the two vectors in the presence of the two drugs. Thus, high production of a chaperonin fusion protein is achieved by expressing these genes in the transformant.

The hosts producing the chaperonin fusion protein or the chaperonin-target protein include, but is not limited to, bacteria such as E. coli, other prokaryotic cells, yeasts, insect cells, animal cells, plant cells, animals, plants, and insects. In particular, bacteria or yeasts are preferable because of low cost for culturing, a reduced number of days for culturing, and easy operations for culturing. Further, the chaperonin fusion protein can also be expressed as a soluble protein in a cell-free translation system using such as an extract from bacteria or eukaryotes (Spirin, A. S., 1991, Science 11, 2656-2664: Falcone, D. et al., 1991, Mol. Cell. Biol. 11, 2656-2664).

The chaperonin-target protein complex is purified according to the following procedure, for example. After the chaperonin fusion protein and the target protein are expressed in the host, the cells are collected and disrupted to recover a supernatant. Next, the chaperonin-target protein is precipitated at about 40% saturation of ammonium sulfate. The precipitated fraction is recovered, dissolved in an appropriate buffer and subjected to gel filtration, hydrophobic chromatography, ion-exchange chromatography, and the like to recover fractions containing the chaperonin-target protein complex, whereby the purified chaperonin-target protein is obtained.

The chaperonin-target protein complex obtained by the invention is applicable for variety of uses. One of them is stabilization of proteins. Generally, a protein is affected by heat and the like, whereby its higher-ordered structure is broken to have a hydrophobic core accommodated in the higher-ordered structure exposed on the surface of the protein. The denatured protein because of interaction via the hydrophobic core forms an irreversible aggregate, thereby inhibiting spontaneous recovery of a native structure. By use of the chaperonin-target protein in the invention, the target protein is accommodated in the chaperonin ring, so that an irreversible aggregate formation is not caused even if the high-structure is broken to have the hydrophobic core exposed on the surface of the protein. A chaperonin essentially has a function of assisting protein folding, so as to have also an inhibitory effect on denaturation itself of a target protein. If a target protein is an enzyme which employs a low molecular weight compound as a substrate, which compound is adapted to pass between a cavity in a chaperonin and the outside, a target protein is ensured its stability by being accommodated in the chaperonin and fully exerts innate characteristic of a protein such as an enzyme reaction.

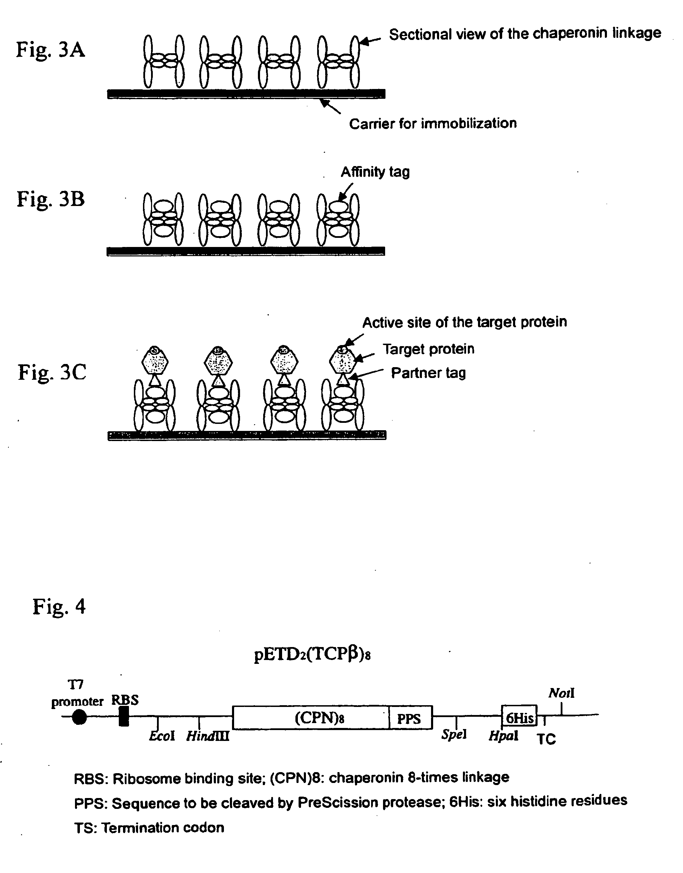

The chaperonin-target protein in the invention is applicable to immobilize the target protein on a carrier. Generally, a chaperonin recognizes a hydrophobic surface of a denatured protein, so that a top part of a chaperonin ring forms a hydrophobic cluster. Consequently, if and when the chaperonin is acted with a support made of hydrophobic materials such as polystyrene, the top part of the chaperonin ring is bound to the surface of the support (Fig. 3A). A chaperonin has two rings arranged symmetrically on either side of an equatorial plane, whereby a plane of first ring is bound to a support with the consequence that a top part of second ring faces in a vertical direction. Further, two-dimensional crystallization of the chaperonin on the support arranges the chaperonin more precisely on the support. In this way, all the chaperonins contacting with the support are controlled in an even direction, and then immobilized. In the same way, even if a chaperonin-affinity tag fusion protein having an affinity tag in a ring is acted with a support, it is immobilized evenly in a vertical direction on the support like the chaperonin-target protein complex in the invention (Fig. 3B). The target protein includes a partner tag having an affinity for an affinity tag, so that the target protein is immobilized on a support via the chaperonin fusion protein by means of the specific affinity between the two tags (Fig. 3C). As just described, the immobilized target protein does not get denatured resulting from a change in a higher-ordered structure by means of hydrophobic interaction with a carrier in comparison with a protein directly immobilized to a carrier by the conventional means. By means of an immobilizing method in the present invention, the affinity tag is oriented evenly via the chaperonin, so that the target protein is immobilized in order. Thus, an active site of the target protein faces a reactive interface, thereby exerting efficiently a characteristic of the immobilized target protein (Fig. 3-3). The immobilizing method in the present invention has an advantage of the target protein being protected, being resistant to denaturation, and ensuring high stability because a part of the immobilized protein or a whole of the molecule is accommodated in the chaperonin ring.

An X-ray crystallographic analysis of a protein is further performed using the chaperonin-target protein complex in the present invention. The invention is based on a concept of a chaperonin as a molecular container for crystallographic analysis. In order to crystallize a protein, it is originally supposed to examine a condition for crystallization because nature of the molecular surface is different depending on individual proteins. However, by means of a method of analyzing a structure in the present invention, the analysis covers a complex accommodating whole of a target protein in a chaperonin molecule, and whereby a condition for analyzing the complex is controlled by nature of the molecular surface of the chaperonin, not by nature of the target protein. Consequently, any protein, if only be accommodated in the molecule of the chaperonin, can be crystallized under a uniform condition for crystallizing a chaperonin and be subjected to an X-ray analysis. The obtained crystal is analyzed by means of a character X-ray generated upon making an electron collide with a copper or molybdenum or a synchrotron radiation. An intensity measurement of an X-ray diffraction is performed by an X-ray film or a two-dimensional detector such as an imaging plate. A crystal is rotated with the X-ray irradiated to generate a number of diffraction lines. In the case of the imaging film, a recorded diffraction pattern is scanned to be digitalized. According to the method of analyzing the structure in the invention, any protein is crystallized under a uniform condition, so as to speed up of a structural analysis.

Further, the chaperonin-target protein complex in the invention can be used for preparing a sustained-release formulation of a protein drug. More specifically, a chaperonin-target protein complex wherein the target protein is an interferon, an antibody, a cytotoxic factor, or the like is formed and accommodated in the molecule of the chaperonin to give the formulation. Many protein drugs described above have extremely short residence time in vivo with the consequence that they may be decomposed by proteases in the blood before arrival to an affected area. Meanwhile, these proteins act in not only the affected area but also normal body tissues, resulting in a higher incidence of unexpected side effects. As in the present invention, a chaperonin coats a physiologically active protein, thereby protecting the target protein from being decomposed by proteases and also protecting normal cell tissues from being acted in. When the sustained-release formulation in the invention flowing in the blood approaches a specific affected area, proteases produced at the area decompose the chaperonin and release the physiologically active protein accommodated therein to exert the drug efficacy. In the case of giving the present invention to host animals, it is necessary to use a chaperonin derived from the animals to prevent from immune reaction. Especially, in order to give the invention to human, it is preferable to use a chaperonin derived from human such as CCT (chaperonin containing t-complex polypeptide 1; Kubota, H., Eur. J. Biochem., 230, 3, 1995).

Still further, the chaperonin-target protein complex in the invention is applied as an immunogen to produce an antibody against the target protein. More specifically, when the protein is inoculated to an animal as an immunogen in order to produce an antibody against a target protein, the target protein may be rapidly decomposed in the blood of the inoculated animal before an immune response is induced. However, using the chaperonin-target protein in the invention, the target protein is accommodated in the chaperonin to maintain a residence time in vivo as an antigen. Consequently, an immune response is carried out against the target protein more efficiently. The use of a chaperonin derived from a living body other than the inoculated animal expects also an adjuvant effect. Herein, in order to control a speed to decompose the chaperonin-target protein complex by proteases, a protease recognition site may be arranged at a linking site between the chaperonin subunits or a linking site between the target antigen and the chaperonin subunit. Animals immunized with the chaperonin-target protein complex include mouse, rat, hamster, marmot, rabbit, dog, sheep, goat, horse, pig, chicken, and monkey.

After immunization of an animal with the chaperonin-target protein complex in the invention as an immunogen, an antibody is obtained in the known way. For example, a polyclonal antibody is obtained from an antiserum of an immunized animal. A monoclonal antibody is obtained from hybridoma culture wherein the hybridoma producing an antibody recognizing a target protein is selected from cells made by cell fusion of an antibody-producing cell and a myeloma.

These procedures are performed according to the generally known methods such as the method developed of Kohler and Milstein (Kohler, G. and Milstein, C., Nature 256, 459, 1975).

### EXAMPLES

Hereinafter, this invention is described in more detail by reference to the Examples, but this invention is not limited to the Examples.

### Example 1

### (Construction of an expression vector for Thermococcus strain KS-1 chaperonin β subunit linkage)

In order to prepare a double-stranded DNA, a linker F 1 as shown in SEQ ID No. 9 and a linker R1 as shown in SEQ ID No. 10 that are complementary nucleotide sequences were annealed. The double-stranded DNA includes an NcoI site, an XhoI site, a nucleotide sequence that is translated to be converted to a PreScission protease site, an SpeI site, an HpaI site, a nucleotide sequence that is translated to be converted to a histidine tag, and a termination codon. The double-stranded DNA was treated with NcoI/XhoI, and ligated to pET21d (Novagen) treated in advance with the restriction enzymes. A plasmid obtained thereby was designated pETD₂.

Meanwhile, a chaperonin β subunit (TCPβ) gene as shown in SEQ ID No. 1 was cloned by PCR (Polymerase chain reaction) using genomic DNA of Thermococcus strain KS-1 as a template. The genomic DNA of Thermococcus strain KS-1 was prepared by a phenol/chloroform treatment and an ethanol precipitation method using pellets recovered from suspension of strains (JCM No. 1 11816) obtained from RIKEN. PCR using the genomic DNA as a template and TCPβ F 1 primer as shown in SEQ ID No. 11 and TCPβ R1 primer as shown in SEQ ID No. 12 as a primer set was carried out, whereby a DNA fragment containing a TCPβ gene as shown in SEQ ID No. 1 was amplified. Herein, a Bg1II site and a BamHI site derived from the primers were introduced into each end of the amplified DNA fragment. pT7(TCPβ) was prepared by introduction of the amplified DNA fragment into pT7BlueT vector by TA cloning. As a result of determination of a nucleotide sequence of the DNA fragment introduced into the pT7(TCPβ), it was the same as a nucleotide sequence as shown in SEQ ID No. 1. Next, the pT7(TCPβ) was treated with restriction enzymes Bg1II and BamHI to recover a DNA fragment containing a TCPβ gene. The DNA fragment was introduced into a plasmid pETD₂ cleaved in advance by BamHI to give pETD₂(TCPβ)₁. Herein, the DNA fragment was linked in such a direction that the TCPβ gene in the DNA fragment was translated properly as TCPβ by the promoter. Further, the DNA fragment containing the TCPβ gene wherein the fragment was recovered by the Bg1II/BamHI treatment was introduced into the pETD₂(TCPβ)₁ treated in advance with BamHI to give pETD₂(TCPβ)₂. Herein, the TCPβ is linked in the same direction as described just above. In the same way, the pETD₂(TCPβ)₂ was treated with restriction enzymes Bg1II and BamHI, so that DNA fragments each containing a (TCPβ)₂ gene were recovered. The DNA fragments were introduced into the pETD₂(TCPβ)₂ treated in advance with BamHI to give ₂(TCPβ)₄ wherein four TCPβ genes were linked one another in the same direction.

As well, in order to recover DNA fragments each containing a (TCPβ)₄ gene, the pETD₂(TCPβ)₄ was treated with restriction enzymes Bg1II and BamHI. The DNA fragments were introduced into pETD₂(TCPβ)₄ treated in advance with BamHI to give pETD₂(TCPβ)₈ wherein eight TCPβ genes were linked one another in the same direction. Figure 4 illustrates a structure of the pETD₂(TCPβ)₈. That is, the pETD₂(TCPβ)₈ includes T7 promoter, and downstream thereof, a ribosome binding site (RBS), (TCPβ)₈ genes wherein eight genes encoding TCPβ is arranged in tandem (represented as (CPN)₈ in Fig. 4), a recognition sequence of PreScission protease, a 6His gene encoding six histidine residues, and a termination codon. Further, it is possible to introduce a gene encoding any affinity tag between the SpeI site and the HpaI site so as to express a fusion protein composed of (TCPβ)₈ and the affinity tag.

### Example 2

### (Construction of an expression system for a fusion protein composed of a β subunit 8-times linkage and protein A)

On the other hand, in order to clone a protein A gene as an affinity tag, the protein A gene as shown in SEQ ID No. 2 was cloned by PCR using genomic DNA of Staphylococcus aureus as a template. The genomic DNA of Staphylococcus aureus was prepared in a manner similar to Example 1 using pellets recovered from suspension of strains (DSM 20231) obtained from DSM in Germany. Pro-F1 primer as shown in SEQ ID No. 13 and pro-R1 primer as shown in SEQ ID No. 14 were used as a primer set for PCR. Herein, a SpeI site and an HpaI site were provided respectively at the pro-F1 primer and the pro-R1 primer. As well as Example 1, an amplified DNA was introduced into pT7BlueT vector by TA cloning, with the result of confirmation of its nucleotide sequence being the same as a nucleotide sequence as shown in SEQ ID No. 2. Next, a DNA fragment containing a protein A gene was cleaved and recovered by SpeI/HpaI treatment. The DNA fragment was introduced into pETD₂(TCPβ)₈ treated in advance with SpeI/HpaI, so that an expression vector for a fusion protein composed of a TCPβ 8-times linkage and proton A, pETD₂(TCPβ)₈-ptnA was constructed.

### Example 3

### (Expression and Purification of a chaperonin β subunit 8-times linkage-protein A fusion protein)

The pETD₂(TCPβ)₈-ptnA was introduced into Escherichia coli strain BL21 (DE3) to give a transformant. In order to express a chaperonin subunit 8-times linkage (TCPβ)₈, the transformant was cultured at 35°C in 2xYT medium (Bacto-trypton 16 g, Yeast extract 10 g, and NaCl 5 g/L) containing carbenichillin (100 µg/mL), whereupon IPTG was added so as to get a final concentration of 1 mM at the point when OD600 reached 0.7. After the transformant was cultured for about 16 hours further, the cells were harvested and disrupted by sonication to recover supernatant with centrifugation.

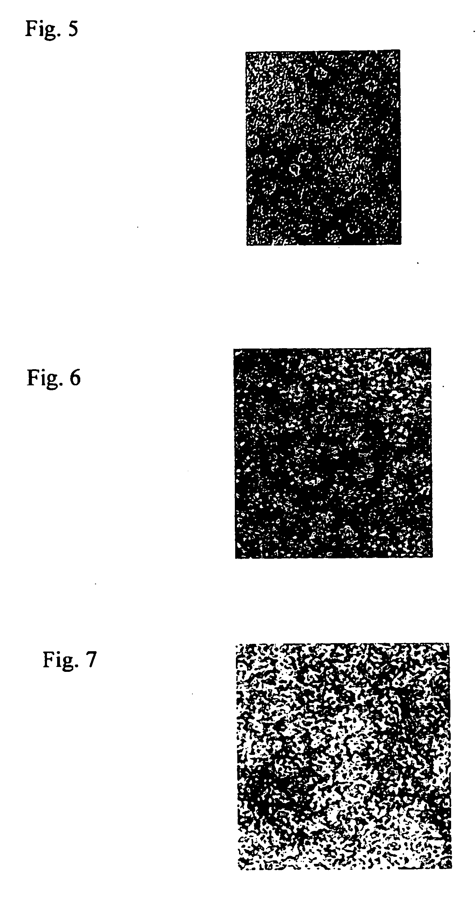

The supernatant was subjected to a nickel chelating column equilibrated in advance with A solution (25 mM Tris-HCl/0. 5 M NaCl/1 mM imidazole (pH 7.0)), whereupon a chaperonin β subunit 8-times linkage-protein A fusion protein was eluted by a linear gradient using B solution (25 mM Tris-HCl/0.5M NaCl/100 mM imidazole (pH 7.0)). The eluted fraction was subjected to a DEAE Toyopearl column (16 mm x 60 cm) equilibrated in advance with a 25 mM HEPES-KOH buffer (pH 6.8), whereupon a fusion protein was eluted by a linear gradient using B solution (25 mM HEPES-KOH/0. 5 M NaCl buffer (pH 6.8)). In the last place, the fraction containing the chaperonin fusion protein was concentrated to be subjected to a HiLoad 26/60 Superdex 200pg column (26 mm x 60 cm) equilibrated in advance with a 100 mM phosphate buffer (pH 7.0) containing 0. 15 M NaCl, whereupon a chaperonin β subunit 8-times linkage-protein A fusion protein was eluted by the buffer. As a result of observation of the purified sample by a negative straining method with 0.2% uranyl acetate under a transmission electron microscope, a ring structure unique to a chaperonin had been formed (Fig. 5).

### Example 4

### (Preparation of a protein complex)

0.6 mg of the chaperonin-protein A fusion protein purified in Example 3 and 0.2 mg of IgG derived from mice were mixed in 0.5 mL of solution and incubated at 30°C for 2 hours. After the reaction was finished, as a result of observation by a negative straining method with 0.2% uranyl acetate under a transmission electron microscope, a ring structure unique to a chaperonin had been formed and antibodies had been accommodated in the ring (Fig. 6).

### Example 5

### (Construction of an expression system for a fusion protein composed of a chaperonin β subunit 8-times linkage and ZZ-tag)

A region of protein A bound to Fc region of IgG is designated ZZ-tag. The ZZ-tag was tried to be fused as the affinity tag with the chaperonin β subunit 8-times linkage. More specifically, PCR using pEZZ 18 protein A fusion vector (Amersham Bioscience) as a template and ZZ-F primer as shown in SEQ ID No. 15 and ZZ-R primer as shown in SEQ ID No. 16 as a primer set was carried out, whereby a DNA fragment containing a ZZ-tag gene as shown in SEQ ID No. 17 was amplified. Herein, the ZZ-F primer has a BamHI site and the ZZ-R primer has a Bg1II site. The PCR product was recovered by use of agarose electrophoresis, and then digested with BamHI/Bg1II. Meanwhile, the digestion product was introduced into pETD₂(TCPβ)₈ treated in advance with BamHI, so that an expression vector for a fusion protein composed of (TCPβ)₈ and the ZZ-tag, pETD₂(TCPβ)₈-ZZ was constructed.

### Example 6

### (Co-expression of a chaperonin-S protein fusion protein and a GFP-S tag)

In order to express a fusion protein composed of (TCPβ)₈ and S-protein, an S-protein gene derived from Bovine as shown in SEQ ID No. 3 was cloned by PCR using Bovine cDNA library (DupLEX-AcDNALibrary, Origene) as a template. Spro-F1 primer as shown in SEQ ID No. 18 and Spro-R1 primer as shown in SEQ ID No. 19 were used as a primer set for PCR. Herein, an SpeI site and an SmaI site were provided respectively at the Spro-F1 primer and the Spro-R1 primer. As well as Example 1, then amplified DNA was introduced into pT7BlueT vector by TA cloning, with the consequence of confirmation of its nucleotide sequence being the same as a nucleotide sequence as shown in SEQ ID No. 3. Next, the plasmid was digested with SpeI/SmaI, whereby a DNA fragment containing an S-protein gene was recovered. The DNA fragment was introduced intro pETD₂(TCPβ)₈ treated in advance with SpeI/HpaI, so that an expression vector for a fusion protein composed of (TCPβ)₈ and S-protein, pETD₂(TCPβ)₈-Sptn was constructed.

Meanwhile, aside from above, as to DNA encoding Green Fluorescent Protein (GFP), GFP expression unit containing a GFP gene and a T7 promoter upstream thereof was cloned by PCR using pQBI T7 sgGFp vector (Funakoshi) as a template. GFP-stagF1 primer as shown in SEQ ID No. 20 and GFP-stagR1 primer as shown in SEQ ID No. 21 were used as a primer set for PCR. Herein, an SphI site and an XhoI site were provided respectively upstream of the T7 promoter of the amplification product and downstream of the GFP gene. An amplified DNA fragment was purified by digestion with SphI/XhoI. Next, an oligonucleotide that becomes S-tag by translation as shown in SEQ ID No. 22 and an oligonucleotide that is its complementary strand as shown in SEQ ID No. 23 were synthesized. These oligonucleotides were annealed to give a double-stranded DNA. Herein, the double-stranded DNA has an XhoI site and a BamHi site on each end. The double-stranded DNA and the digestion product with the restriction enzymes were mixed and ligated in such a manner as arranged in GFP-Stag gene order in pACYC184 plasmid DNA (Wako Pure Chemical Industries) digested in advance with SphI/BamHI. An expression plasmid of GFP obtained in this way was designated pACGFPstag.

### Example 7

### (Co-expression of a chaperonin-S protein fusion protein and a GFP-S tag)

Both of the plasmids pETD₂(TCPβ)₈-Sptn and pACGFPstag obtained in Example 6 were added into a competent cell of Escherichia coli strain BL21 (DE3) to give a transformant, which was cultured on an agar medium containing 100 µg/mL ampicillin and 100 µg/mL chloramphenicol. A colony obtained thereby was inoculated to 700 mL of 2xYT medium containing 100 µg/mL ampicillin and 100 µg/mL chloramphenicol. After rotary culture (100 rpm) at 35°C, IPTG was added so as to get a final concentration of 1 mM at the point when OD600 reached 0.7, thereby inducing an expression of a chaperonin-S protein fusion protein and a GFP-s tag. Cells were harvested by centrifugation (10,000 rpm x 10 minutes), suspended in 20 mL of 25 mM HEPES buffer (pH 6.8) containing 1 mM EDTA, and cryopreserved at -20°C. After the cell suspension was melted, supernatant fraction was obtained by means of disruption by sonication.

The obtained supernatant was added onto a butyl Toyopearl (Tosoh) equilibrated in advance with 50 mM phosphate buffer (pH 7.0) containing 1 M ammonium sulfate, whereupon a fraction containing the chaperonin-S protein fusion protein was recovered by a linear gradient using B solution (50 mM phosphate buffer (pH 7.0)). Next, the fraction was subjected to anion-exchange chromatography and gel filtration in the same way as Example 3, whereupon a fraction wherein the chaperonin fusion protein was eluted was recovered. Irradiated with ultra violet, the obtained fraction emitted fluorescein of GFP. As a result of observation of the obtained fraction by a negative straining method with 0.2% uranyl acetate under a transmission electron microscope, a ring structure unique to a chaperonin had been formed and GFPs were accommodated in the ring (Fig. 7)

### Example 8

### (Immobilization of IgG on a carrier via a chaperonin-protein A fusion protein)

50 µL of the chaperonin-protein A fusion protein (1 mg/mL) obtained in Example 3 was added to a polystyrene 96-well microtiterplate, followed by incubation at 30°C for 3 hours, whereby the fusion protein was immobilized on the plate. After being washed with PBS, the plate was blocked with 50% Block Ace (Dainippon Pharmaceutical), and washed again with PBS. Thereafter, 50 µL of 0.1 mg/mL mouse monoclonal anti-HBs antibody was added to the plate, followed by incubation at 30°C for 2 hours, whereby the mouse monoclonal antibody was immobilized via the chaperonin-protein A fusion protein. After free mouse monoclonal antibody was removed by washing with PBS, 100 µL of PBS was added, followed by incubation at 50°C.

A remaining activity of the antibody was evaluated by use of sandwich ELISA. More specifically, 50 µL of 100 µg/mL HBs antigen was added to the plate on which the mouse monoclonal antibody was immobilized, and then sandwiched between the mouse monoclonal antibody and a rabbit anti-HBs antibody after the plate was washed with PBS. The rabbit anti-HBs antibody bound thereto was detected by use of an anti-rabbit IgG antibody conjugated with peroxidase with 2,2'-azido-di (3-ethyl-benzthiazoline-6-sulphonate) as a substrate. Aside from this, an experiment for comparing immobilization of a mouse monoclonal antibody directly on a plate without the chaperonin-protein A fusion protein was evaluated as well.

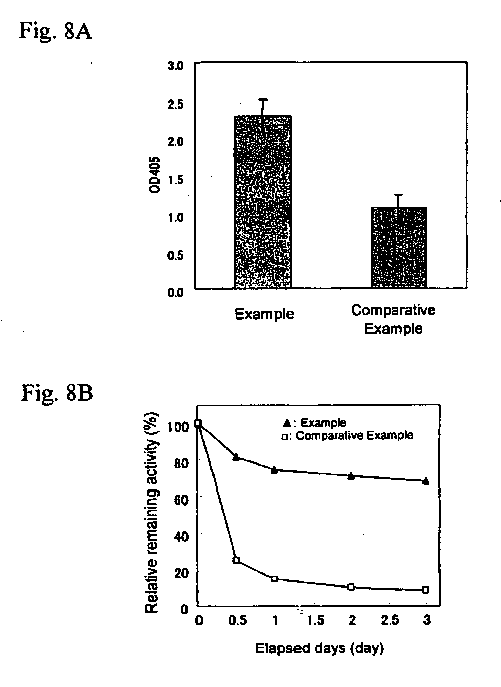

Figure 8A shows a graph for comparing an antibody activity immediately following by immobilization of the mouse monoclonal antibody. Figure 8B shows a graph for comparing a heat stability of the immobilized mouse monoclonal antibody. In consequence of the comparisons, IgG immobilized via the chaperonin-protein A fusion protein had a higher antibody binding ability than IgG directly immobilized at the point immediately after immobilization. Further, IgG immobilized via the chaperonin-protein A fusion protein had higher heat stability than IgG directly immobilized.

## Claims

1. A chaperonin-target protein complex, comprising:
a fusion protein comprising a chaperonin subunit and an affinity tag linked to the chaperonin subunit via a peptide bond; and
a target protein for which the affinity tag shows a specific affinity, wherein the target protein is bound to the affinity tag by means of the specific affinity, thereby forming a chaperonin ring structure consisting of a plurality of chaperonin subunits and wherein the target protein is accommodated in the chaperonin ring structure of the fusion protein.

2. The chaperonin-target protein complex as defined in claim 1, wherein the fusion protein comprises one chaperonin subunit, and wherein the affinity tag is linked via the peptide bond to the N-terminus and/or the C-terminus of the chaperonin subunit.

3. The chaperonin-target protein complex as defined in claim 1, wherein the fusion protein comprises a chaperonin subunit linkage composed of 2 to 20 chaperonin subunits serially linked to one another, and wherein the affinity tag is linked to at least one site selected from a group consisting of the N-terminus of the chaperonin subunit linkage, the C-terminus of the chaperonin subunit linkage, and a linking site of the chaperonin subunits.

4. The chaperonin-target protein complex as defined in claim 1, 2, or 3, wherein the ratio of the number of the chaperonin subunits to the number of the affinity tag is in the range of from 1 : 2 to 9 : 1.

5. The chaperonin-target protein complex as defined in claim 3 or 4, being provided with a sequence to be cleaved by a site-specific protease between the subunits of the chaperonin subunit linkage.

6. The chaperonin-target protein complex as defined in one of claims 1-5, having two chaperonin rings non-covalently associated on each other's ring plane so as to form a two-layer chaperonin ring structure.

7. The chaperonin-target protein complex as defined in claim 6, having two chaperonin rings non-covalently associated on each other's ring plane or on each other's side so as to form a fibrous chaperonin ring structure.

8. The chaperonin-target protein complex as defined in one of claims 1 to 7, wherein the living thing from which the chaperonin is derived is one selected from a group consisting of bacteria, archaea, and eukaryotes.

9. The chaperonin-target protein complex as defined in one of claims 1 to 8, wherein the affinity tag is one selected from a group consisting of an antibody, streptoavidin, protein A, protein G, protein L, S peptide, S-protein, and a partial fragment thereof.

10. The chaperonin-target protein complex as defined in one of claims 1 to 9, wherein the target protein is a polypeptide including a fragment of six or more amino acid residues, and includes a partner tag having a specific affinity for the affinity tag.

11. The chaperonin-target protein complex as defined in one of claims 1 to 10, wherein the target protein is one selected from a group consisting of a heavy chain of an antibody and a light chain of an antibody and a polypeptide including a fragment of 6 or more amino acid residues thereof, and includes a partner tag having a specific affinity for the affinity tag.

12. The chaperonin-target protein complex as defined in one of claims 1 to 10, wherein the target protein includes one selected from a group consisting of a virus antigen, a seven transmembrane receptor, a cytokine, a protein kinase, a phosphoprotein phosphatase, and a partial fragment thereof.

13. A method of producing the chaperonin-target protein complex as defined in one of claims 1 to 12, the method comprising steps of:
mixing the fusion protein composed of the chaperonin subunit and the affinity tag with the target protein including the partner tag having an affinity for the affinity tag; and
binding the fusion protein to the target protein by means of a specific affinity between the affinity tag and the partner tag.

14. A method of producing the chaperonin-target protein complex as defined in one of claims 1 to 12, wherein the partner tag having a specific affinity for the affinity tag is a polypeptide, and the method comprising a step of transcribing and translating in the same host both of a gene containing a gene encoding the chaperonin subunit and a gene encoding the affinity tag and a gene containing a gene' encoding the target protein and a gene encoding the partner tag, thereby binding the target protein to the chaperonin fusion protein.

15. A method of producing the chaperonin-target protein complex as defined in one of claims 1 to 12, wherein the partner tag having a specific affinity for the affinity tag is a polypeptide, and the method comprising a step of transcribing and translating in the same host three genes consisting of a gene containing a gene encoding the chaperonin subunit and a gene encoding the affinity tag, a gene containing a gene encoding the target protein and a gene encoding the partner tag, and a gene encoding the chaperonin subunit or its linkage, thereby binding the target protein, the chaperonin fusion protein, and the chaperonin subunit or its linkage.

16. The method as defined in claim 14 or 15, wherein the protein complex is produced in a host selected from a group consisting of bacteria, yeasts, animal cells, plant cells, insect cells, animals, plants, and insects.

17. The method as defined in claim 14 or 15, wherein the protein complex is produced in a cell-free translation system.

18. A method of stabilizing the target protein, the method comprising a step of binding the target protein via the affinity tag to the fusion protein included in the chaperonin-target protein complex as defined in one of claims 1 to 9 to have the target protein be accommodated in the chaperonin ring structure.

19. A method of immobilizing the target protein, the method comprising steps of:
immobilizing on a carrier for immobilization the fusion protein included in the chaperonin-target protein complex as defined in one of claims 1 to 9, and
binding the target protein via the affinity tag to the fusion protein to have the target protein be accommodated in the chaperonin ring.

20. A method of analyzing the structure of the target protein, the method comprising steps of:
crystallizing a protein complex obtained by accommodating the target protein bound via the affinity tag to the fusion protein included in the chaperonin-target protein complex as defined in one of claims 1 to 9, and
obtaining information about three-dimensional structure of the target protein from an X-ray diffraction image obtained by irradiating a crystal obtained by the crystallizing.

21. A sustained-release formulation for a physiologically active protein or a low molecular weight drug, wherein the physiologically active protein or the low molecular weight drug is accommodated in the fusion protein included in the chaperonin-target protein complex as defined in one of claims 1 to 9 via the partner tag for the affinity tag.

22. The sustained-release formulation as defined in claim 21, wherein the chaperonin is derived from human.

23. A method of producing an antibody against a target antigen protein, the method comprising steps of:
binding the target antigen protein to the fusion protein included in the chaperonin-target protein complex as defined in one of claims 1 to 9, and
immunizing a non-human animal therewith as an immunogen.

24. The chaperonin-target protein complex as defined in one of claims 1 to 12 for use as an immunogen to produce an antibody against the target protein.

25. Use of a chaperonin-target protein complex as defined in one of claims 1 to 12 for immunizing an animal.

## Patentansprüche

1. Chaperonin-Zielproteinkomplex umfassend:
ein Fusionsprotein, das eine Chaperonin-Untereinheit und einen Affinitätsliganden, der über eine Peptidbindung mit der Chaperonin-Untereinheit verbunden ist, umfasst; und
ein Zielprotein, für welches der Affinitätsligand eine spezifische Affinität aufweist, wobei das Zielprotein durch die spezifische Affinität an den Affinitätsliganden gebunden ist, wodurch eine Chaperonin-Ringstruktur ausgebildet wird, die aus einer Vielzahl an Chaperonin-Untereinheiten besteht, und wobei das Zielprotein in der Chaperonin-Ringstruktur des Fusionsproteins untergebracht ist.

2. Chaperonin-Zielproteinkomplex wie in Anspruch 1 definiert, wobei das Fusionsprotein eine Chaperonin-Untereinheit umfasst und wobei der Affinitätsligand über die Peptidbindung mit dem N-Terminus und/oder dem C-Terminus der Chaperonin-Untereinheit verbunden ist.

3. Chaperonin-Zielproteinkomplex wie in Anspruch 1 definiert, wobei das Fusionsprotein eine Chaperonin-Untereinheit-Verbindung umfasst, die aus 2 bis 20 Chaperonin-Untereinheiten besteht, die seriell miteinander verbunden sind, und wobei der Affinitätsligand mit wenigstens einer Stelle verbunden ist, die aus der Gruppe bestehend aus dem N-Terminus der Chaperonin-Untereinheit-Verbindung, dem C-Terminus der Chaperonin-Untereinheit-Verbindung und einer Bindungsstelle der Chaperonin-Untereinheiten, ausgewählt ist.

4. Chaperonin-Zielproteinkomplex wie in Ansprüchen 1, 2 oder 3 definiert, wobei das Verhältnis der Anzahl an Chaperonin-Untereinheiten zu der Anzahl des Affinitätsliganden im Bereich zwischen 1 : 2 bis 9 : 1 liegt.

5. Chaperonin-Zielproteinkomplex wie in Anspruch 3 oder 4 definiert, der mit einer Sequenz zwischen den Untereinheiten der Chaperonin-Untereinheit-Verbindung ausgestattet ist, die durch eine ortsspezifische Protease gespalten werden kann.

6. Chaperonin-Zielproteinkomplex wie in einem der Ansprüche 1 bis 5 definiert, mit zwei Chaperonin-Ringen, die nicht-kovalent untereinander über ihre Ringfläche verbunden sind, so dass sie eine zweischichtige Chaperonin-Ringstruktur ausbilden.

7. Chaperonin-Zielproteinkomplex wie in Anspruch 6 definiert, mit zwei Chaperonin-Ringen, die nicht-kovalent untereinander über ihre Ringfläche oder durch ihre Flanken so verbunden sind, dass sie eine fadenartige Chaperonin-Ringstruktur ausbilden.

8. Chaperonin-Zielproteinkomplex, wie in einem der Ansprüche 1 bis 7 definiert, wobei das Lebewesen, von welchem das Chaperonin stammt, eines ist, das aus der Gruppe bestehend aus Bakterien, Archaea und Eukaryonten ausgewählt ist.

9. Chaperonin-Zielproteinkomplex wie in einem der Ansprüche 1 bis 8 definiert, wobei der Affinitätsligand einer ist, der aus der Gruppe bestehend aus einem Antikörper, Streptavidin, Protein A, Protein G, Protein L, S-Peptid, S-Protein und einem Teilfragment davon ausgewählt ist.

10. Chaperonin-Zielproteinkomplex wie in einem der Ansprüche 1 bis 9 definiert, wobei das Zielprotein ein Polypeptid ist, das ein Fragment von sechs oder mehr Aminosäureresten umfasst und einen Partnerliganden umfasst, der eine spezifische Affinität zu dem Affinitätsliganden aufweist.

11. Chaperonin-Zielproteinkomplex wie in einem der Ansprüche 1 bis 10 definiert, wobei das Zielprotein eines ist, das aus der Gruppe ausgewählt ist, bestehend aus einer schweren Kette eines Antikörpers und einer leichten Kette eines Antikörpers und einem Polypeptid, das ein Fragment von 6 oder mehr Aminosäuren davon umfasst, und das einen Partnerliganden umfasst, der eine spezifische Affinität für den Affinitätsliganden aufweist.

12. Chaperonin-Zielproteinkomplex wie in einem der Ansprüche 1 bis 10 definiert, wobei das Zielprotein eines umfasst, das aus der Gruppe bestehend aus einem Virusantigen, einem Siebentransmembranrezeptor, einem Cytokin, einer Proteinkinase, einer Phosphoproteinphosphatase und einem Teilfragment davon ausgewählt ist.

13. Verfahren zur Herstellung des Chaperonin-Zielproteinkomplexes wie in einem der Ansprüche 1 bis 12 definiert, wobei das Verfahren die Schritte umfasst:
Mischen des Fusionsproteins, das aus der Chaperonin-Untereinheit und dem Affinitätsliganden zusammengesetzt ist, mit dem Zielprotein, das den Partnerliganden, der eine Affinität für den Affinitätsliganden aufweist, umfasst; und
Binden des Fusionsproteins durch die spezifische Affinität zwischen dem Affinitätsliganden und dem Partnerliganden an das Zielprotein.

14. Verfahren zur Herstellung des Chaperonin-Zielproteinkomplexes, wie in einem der Ansprüche 1 bis 12 definiert, wobei der Partnerligand, der eine spezifische Affinität für den Affinitätsliganden aufweist, ein Polypeptid ist und das Verfahren einen Schritt umfasst, in demselben Wirt, sowohl ein Gen zu transkribieren und translatieren, das ein Gen, das für die Chaperonin-Untereinheit kodiert und ein Gen, das für den Affinitätsliganden kodiert, umfasst, als auch ein Gen zu transkribieren und translatieren, das ein Gen, das für das Zielprotein kodiert und ein Gen, das für den Partnerliganden kodiert, umfasst, wodurch das Zielprotein an das Chaperonin-Fusionsprotein gebunden wird.

15. Verfahren zur Herstellung des Chaperonin-Zielproteinkomplexes wie in einem der Ansprüche 1 bis 12 definiert, wobei der Partnerligand, der eine spezifische Affinität für den Affinitätsliganden aufweist, ein Polypeptid ist und das Verfahren einen Schritt umfasst, in demselben Wirt, drei Gene, bestehend aus einem Gen, das ein Gen, das für die Chaperonin-Untereinheit kodiert und ein Gen, das für den Affinitätsliganden kodiert, umfasst, einem Gen, das ein Gen, das für das Zielprotein kodiert und ein Gen, das für den Partnerliganden kodiert, umfasst, und einem Gen, das für die Chaperonin-Untereinheit oder deren Verbindung kodiert, zu transkribieren oder translatieren, wodurch das Zielprotein, das Chaperonin-Fusionsprotein und die Chaperonin-Untereinheit oder deren Verbindung verbunden werden.

16. Verfahren wie in Anspruch 14 oder 15 definiert, wobei der Proteinkomplex in einem Wirt hergestellt wird, der aus einer Gruppe, bestehend aus Bakterien, Hefen, Tierzellen, Pflanzenzellen, Insektenzellen, Tieren, Pflanzen und Insekten, ausgewählt ist.

17. Verfahren wie in Anspruch 14 oder 15 definiert, wobei der Proteinkomplex in einem zellfreien Translationssystem hergestellt wird.

18. Verfahren zur Stabilisierung des Zielproteins, wobei das Verfahren einen Schritt umfasst, in dem das Zielprotein über den Affinitätsliganden an das Fusionsprotein gebunden wird, das in dem Chaperonin-Zielproteinkomplex, wie in einem der Ansprüche 1 bis 9 definiert, umfasst ist, um das Zielprotein in der Chaperonin-Ringstruktur unterzubringen.

19. Verfahren zur Immobilisierung des Zielproteins, wobei das Verfahren die Schritte umfasst:
Immobilisieren des Zielproteins, das in dem Chaperonin-Zielproteinkomplex, wie in einem der Ansprüche 1 bis 9 definiert, umfasst ist, auf einem Träger zur Immobilisierung und
Binden des Zielproteins über den Affinitätsliganden an das Fusionsprotein, um das Zielprotein in den Chaperonin-Ring unterzubringen.

20. Verfahren, um die Struktur des Zielproteins zu untersuchen, wobei das Verfahren die Schritte umfasst:
Kristallisieren eines Proteinkomplexes, der durch Unterbringung des Zielproteins erhalten wird, das über den Affinitätsliganden an das Fusionsprotein gebunden ist, das in dem Chaperonin-Zielproteinkomplex, wie in einem der Ansprüche 1 bis 9 definiert, umfasst ist, und
Gewinnen von Information über die dreidimensionale Struktur des Zielproteins aus einem Röntgenbeugungsbild, das durch Bestrahlung eines Kristalls erhalten wurde, der durch die Kristallisierung erhalten wurde.

21. Retard-Rezeptur für ein physiologisch wirksames Protein oder ein Medikament von geringem Molekulargewicht, wobei das physiologisch wirksame Protein oder das Medikament von geringem Molekulargewicht über den Partnerliganden für den Affinitätsliganden in dem Fusionsprotein untergebracht ist, das in dem Chaperonin-Zielproteinkomplex, wie in einem der Ansprüche 1 bis 9 definiert, umfasst ist.

22. Retard-Rezeptur, wie in Anspruch 21 definiert, wobei das Chaperonin vom Menschen stammt.

23. Verfahren zur Herstellung eines Antikörpers gegen ein Zielantigenprotein, wobei das Verfahren die Schritte umfasst:
Binden des Zielantigenproteins an das Fusionsprotein, das in dem Chaperonin-Zielproteinkomplex, wie in einem der Ansprüche 1 bis 9 definiert, enthalten ist und
Immunisieren eines nicht-menschlichen Tieres damit als Immunogen.

24. Chaperonin-Zielproteinkomplex, wie in einem der Ansprüche 1 bis 12 definiert, zur Verwendung als Immunogen, um einen Antikörper gegen das Zielprotein herzustellen.

25. Verwendung des Chaperonin-Zielproteinkomplexes, wie in einem der Ansprüche 1 bis 12 definiert, für die Immunisierung eines Tieres.

## Revendications

1. Complexe de chaperonine-protéine cible, comprenant:
une protéine de fusion comprenant une sous-unité chaperonine et un marqueur à affinité lié à la sous-unité chaperonine via une liaison peptidique; et
une protéine cible pour laquelle le marqueur à affinité présente une affinité spécifique, tandis que la protéine cible est liée au marqueur à affinité au moyen de l'affinité spécifique, formant ainsi une structure cyclique de chaperonine consistant en une pluralité de sous-unités chaperonine, et tandis que la protéine cible est accommodée dans la structure cyclique de chaperonine de la protéine de fusion.

2. Complexe de chaperonine-protéine cible selon la revendication 1, dans lequel la protéine de fusion comprend une sous-unité chaperonine, et dans lequel le marqueur à affinité est lié via la liaison peptidique à l'extrémité N-terminale et/ou à l'extrémité C-terminale de la sous-unité chaperonine.

3. Complexe de chaperonine-protéine cible selon la revendication 1, dans lequel la protéine de fusion comprend une liaison à sous-unité chaperonine composée de 2 à 20 sous-unités chaperonine liées en série l'une à l'autre, et tandis que le marqueur à affinité est lié à au moins un site choisi dans le groupe consistant en l'extrémité N-terminale de la liaison à sous-unité chaperonine, l'extrémité C-terminale de la liaison à sous-unité chaperonine, et un site de liaison des deux sous-unités chaperonine.

4. Complexe de chaperonine-protéine cible selon la revendication 1, 2 ou 3, dans lequel le rapport du nombre des sous-unités chaperonine au nombre de marqueurs à affinité est dans l'intervalle de 1:2 à 9:1.

5. Complexe de chaperonine-protéine cible selon la revendication 3 ou 4, procuré avec une séquence destinée à être clivée par une protéase spécifique pour un site entre les sous-unités de la liaison à sous-unités chaperonine.

6. Complexe de chaperonine-protéine cible selon l'une des revendications 1-5, ayant deux cycles de chaperonine associés de manière non-covalente sur le plan des cycles de chacun d'eux pour former une structure à cycle chaperonine à deux couches.

7. Complexe de chaperonine-protéine cible selon la revendication 6, ayant deux cycles chaperonine associés de manière non-covalente sur le plan des cycles de chacun d'eux ou sur le côté de chacun d'eux de manière à former une structure cyclique de chaperonine fibreuse.

8. Complexe de chaperonine-protéine cible selon l'une des revendications 1 à 7, dans lequel le matériel vivant duquel la chaperonine est dérivée est un matériel choisi dans le groupe consistant en les bactéries, archaea et eucaryotes.

9. Complexe de chaperonine-protéine cible selon l'une des revendications 1 à 8, dans lequel le marqueur à affinité est un marqueur choisi dans le groupe consistant en anticorps, streptavidine, protéine A, protéine G, protéine L, peptide S, protéine S, et un fragment partiel de ceux-ci.

10. Complexe de chaperonine-protéine cible selon l'une des revendications 1 à 9, dans lequel la protéine cible est un polypeptide incluant un fragment de six ou plus de six résidus d'acides aminés, et comporte un marqueur partenaire ayant une affinité spécifique pour le marqueur à affinité.

11. Complexe de chaperonine-protéine cible selon l'une des revendications 1 à 10, dans lequel la protéine cible est une protéine choisie dans le groupe consistant en une chaîne lourde d'un anticorps et une chaîne légère d'un anticorps et un polypeptide comportant un fragment de 6 ou plus de 6 résidus d'acides aminés de celui-ci, et comporte un marqueur partenaire ayant une affinité spécifique pour le marqueur à affinité.

12. Complexe de chaperonine-protéine cible selon l'une des revendications 1 à 10, dans lequel la protéine cible comporte une protéine choisie dans le groupe consistant en un antigène viral, un récepteur sept transmembranaire, une cytokine, une protéine kinase, une phosphoprotéine phosphatase, et un fragment partiel de ceux-ci.

13. Procédé pour la production du complexe de chaperonine-protéine cible selon l'une des revendications 1 à 12, le procédé comprenant les étapes de:
mélange de la protéine de fusion composée de la sous-unité chaperonine et du marqueur à affinité avec la protéine cible comportant le marqueur partenaire ayant une affinité pour le marqueur à affinité; et
liaison de la protéine de fusion à la protéine cible au moyen d'une affinité spécifique entre le marqueur à affinité et le marqueur partenaire.

14. Procédé pour la production du complexe de chaperonine-protéine cible selon l'une des revendications 1 à 12, dans lequel le marqueur partenaire ayant une affinité spécifique pour le marqueur à affinité est un polypeptide, et le procédé comprenant une étape de transcription et de traduction dans le même hôte à la fois d'un gène contenant un gène codant pour la sous-unité chaperonine et un gène codant pour le marqueur à affinité, et d'un gène contenant un gène codant pour la protéine cible et un gène codant pour le marqueur partenaire, liant ainsi la protéine cible à la protéine de fusion de chaperonine.

15. Procédé pour la production du complexe de chaperonine-protéine cible selon l'une des revendications 1 à 12, dans lequel le marqueur partenaire ayant une affinité spécifique pour le marqueur à affinité est un polypeptide, et le procédé comprenant une étape de transcription et de traduction dans le même hôte de trois gènes consistant en un gène contenant un gène codant pour la sous-unité chaperonine et un gène codant pour le marqueur à affinité, un gène codant pour la protéine cible et un gène codant pour le marqueur partenaire, et un gène codant pour la sous-unité chaperonine ou sa liaison, liant ainsi la protéine cible, la protéine de fusion de chaperonine, et la sous-unité chaperonine ou sa liaison.

16. Procédé selon la revendication 14 ou 15, dans lequel le complexe de protéine est produit chez un hôte choisi dans le groupe consistant en les bactéries, les levures, les cellules animales, les cellules végétales, les cellules d'insectes, les animaux, les plantes et les insectes.

17. Procédé selon la revendication 14 ou 15, dans lequel le complexe de protéine est produit dans un système de traduction exempt de cellules.

18. Procédé pour la stabilisation d'une protéine cible, le procédé comprenant une étape de liaison de la protéine cible via un marqueur à affinité à la protéine de fusion comprise dans le complexe de chaperonine-protéine cible selon l'une des revendications 1 à 9 pour rendre la protéine cible accommodée dans la structure cyclique de chaperonine.

19. Procédé pour l'immobilisation d'une protéine cible, le procédé comprenant les étapes de:
immobilisation sur un support pour immobilisation de la protéine de fusion comprise dans le complexe de chaperonine-protéine cible selon l'une des revendications 1 à 9, et
liaison de la protéine cible via le marqueur à affinité à la protéine de fusion pour rendre la protéine cible accommodée dans le cycle de chaperonine.

20. Procédé pour l'analyse de la structure d'une protéine cible, le procédé comprenant les étapes de:
cristallisation d'un complexe de protéine obtenu par accommodation de la protéine cible liée via le marqueur à affinité à la protéine de fusion incluse dans le complexe de chaperonine-protéine cible selon l'une des revendications 1 à 9, et
obtention de l'information sur la structure tridimensionnelle de la protéine cible à partir d'une image de diffraction aux rayons X obtenue par irradiation d'un cristal obtenu par cristallisation.

21. Formulation à libération prolongée pour une protéine physiologiquement active ou un médicament de faible masse moléculaire, dans laquelle la protéine physiologiquement active ou le médicament de faible masse moléculaire est accommodé dans la protéine de fusion incluse dans le complexe de chaperonine-protéine cible selon l'une des revendications 1 à 9 via le marqueur partenaire pour le marqueur à affinité.

22. Formulation à libération prolongée selon la revendication 21, dans laquelle la chaperonine est dérivée d'un humain.

23. Procédé pour la production d'un anticorps contre une protéine d'antigène cible, le procédé comprenant les étapes de:
liaison de la protéine d'antigène cible à la protéine de fusion incluse dans le complexe de chaperonine-protéine cible selon l'une des revendications 1 à 9, et immunisation d'un animal non-humain avec celui-ci, à titre d'agent immunogène.

24. Complexe de chaperonine-protéine cible selon l'une des revendications 1 à 12 pour utilisation à titre d'agent immunogène pour produire un anticorps contre la protéine cible.

25. Utilisation d'un complexe de chaperonine-protéine cible selon l'une des revendications 1 à 12 pour l'immunisation d'un animal.
